# EUROPEAN PATENT SPECIFICATION

(11) **EP 4 195 952 B1**
(45) Date of publication and mention of the grant of the patent: **30.09.2026**
(21) Application number: 21756009.3
(22) Date of filing: 10.08.2021
(51) Int. Cl.: A23L 31/00, C12N 11/02, C12N 1/14, A01G 18/20, A23J 3/22, A23J 3/20, A23C 19/068, A23L 11/50

(54) **METHOD OF PRODUCING A FUNGUS-BASED FOOD PRODUCT BY PROVIDING A THREE-DIMENSIONAL SCAFFOLD AND A FUNGUS-BASED FOOD PRODUCT OBTAINABLE BY SUCH A METHOD**
VERFAHREN ZUR HERSTELLUNG EINES LEBENSMITTELPRODUKTES AUF PILZBASIS DURCH BEREITSTELLUNG EINES DREIDIMENSIONALEN GERÜSTS UND DURCH EIN SOLCHES VERFAHREN ERHÄLTLICHES LEBENSMITTELPRODUKT AUF PILZBASIS
PROCÉDÉ DE PRODUCTION D'UN PRODUIT ALIMENTAIRE À BASE DE CHAMPIGNONS PAR APPORT D'UN ÉCHAFAUDAGE TRIDIMENSIONNEL ET PRODUIT ALIMENTAIRE À BASE DE CHAMPIGNONS POUVANT ÊTRE OBTENU SELON UN TEL PROCÉDÉ

(30) Priority: 13.08.2020 EP 20190937
(43) Date of publication of application: 21.06.2023
(73) Proprietor: Planted Foods AG, 8310 Kemptthal (CH)
(72) Inventor: RÜHS, Patrick, 8057 Zürich (CH); BÖNI, Lukas, 8057 Zürich (CH); WEMMER, Judith, 8005 Zürich (CH); KOFMEL, Colin, 8406 Winterthur (CH); SAVORANI, Laura Alessia, 8050 Zürich (CH)
(74) Representative: Vossius & Partner Patentanwälte Rechtsanwälte mbB
(86) International application number: PCT/EP2021/072289
(87) International publication number: WO 2022/034092

(56) References cited:
- EP-A1- 2 835 058
- WO-A1-2015/024751
- WO-A1-2020/106743
- WO-A1-2020/164680
- WO-A2-2008/073489
- CN-A- 107 373 382
- CN-A- 111 011 780
- CN-A- 111 374 305
- US-A- 3 885 048
- JAVIER ENRIONE ET AL: "Edible Scaffolds Based on Non-Mammalian Biopolymers for Myoblast Growth", MATERIALS, vol. 10, no. 12, 8 December 2017 (2017-12-08), pages 1404, XP055621815, DOI: 10.3390/ma10121404
- JAYACHANDRAN VENKATESAN ET AL: "Development of Alginate-Chitosan-Collagen Based Hydrogels for Tissue Engineering", JOURNAL OF BIOMATERIALS AND TISSUE ENGINEERING, vol. 5, no. 6, 1 June 2015 (2015-06-01), US, pages 458 - 464, XP055621810, ISSN: 2157-9083, DOI: 10.1166/jbt.2015.1338

## Description

The present invention relates to a method of making a fungus-based food product by providing a three-dimensional edible matrix as a scaffold for fungal growth. Through this fungal growth in the provided three-dimensional edible scaffold, a fibrous fungus-based food product is formed.

Current meat consumption is depleting our natural resources while fuelling climate change (Foley 2011). Our current meat consumption is unsustainable and therefore meat alternatives must be developed to counteract the ever-growing consumption of meat. Current approaches envision the use of meat analogues because such foods allow consumers to adhere to existing cooking recipes and cultural habits. Consequently, alternative food protein sources such as plants, algae, and fungi are becoming increasingly popular as a sustainable solution to replace animal products. Fungi-based meat replacements are intriguingly new meat substitutes as the fungus mycelium, the vegetative part, grows naturally into long, branching fibers resembling the structure of muscle tissue. Additionally, given the naturally occurring aroma components, fungi-based products do not require masking of unpleasant taste as known from plant-based meat alternatives. Current approaches to produce fungi-based meat alternatives often rely on growing mycelium in tanks with nutrients, followed by harvesting and various post-processes including cutting, heat-treating, extruding, mixing with additives, and shaping into food products, as performed by Emergy Foods, Boulder, Colo., Prime Roots, disclosed in WO2007/139321 or as known from Quorn production. Besides requiring intensive post-processing, this approach does not allow to use the full potential of the naturally grown fiber structure. Another approach, disclosed in WO2020/023450, suggests growing a porous mat of fungus mycelium on growth medium, which is in a second step infused with plant-derived proteins, fats, micronutrients and flavoring ingredients by application of vacuum. Thus, the porous mat is processed into a food product resembling animal meat. The described growth of fungal mycelium in free space is restricted in height growth, which limits production of "whole cuts". Also, the fiber structure, direction, and thickness is given by the natural growth pattern of the fungal mycelium in free space, which does not allow to tailor the product texture crucial for food products. Hence, they lack the organoleptic properties associated with familiar food, lack global fiber cohesiveness, bite and juiciness. Although fungi-based food products show potential as highly sustainable animal meat replacement, better solutions are required to tailor the product properties to meet consumer needs.

This need for better solutions is addressed by the embodiments as reflected by the claims.

Thus, in a first aspect, the invention relates to a method of making a fungus-based food product, said method comprising the following steps:
(a) providing a three-dimensional edible scaffold comprising an edible matrix, the three-dimensional edible scaffold being formed by first providing a continuous edible matrix which is then subsequently converted into the three-dimensional scaffold by introducing voids into the matrix by foaming or puncturing or a combination thereof, wherein the edible matrix comprises micro- and/or macronutrients required for fungal mycelium growth, wherein step (a) does not comprise an extrusion process to create multiple filaments or macrostructures;
(b) inoculating the three-dimensional edible scaffold with at least one fungus to generate an inoculated scaffold;
(c) incubating the inoculated scaffold at growth conditions that allow for mycelium growth of the at least one fungus so that the at least one fungus grows through the scaffold as a mycelium to form a fungus-based food product.

Such a method allows to create a fungus-based food product, the texture and flavour of which can be adapted. The method allows, for example, to create a fungus-based food product which can be used as a meat analogue that has:
i) directed and aligned fiber-like texture spanning across the entire food product by mycelium growth;
ii) a meat-like bite created by fungal mycelium growth across the entire food product;
iii) juiciness, chewiness, and elasticity created through the three-dimensional scaffold tailored to achieve the desired textural properties;
iv) shaped as a meat product by using a meat-resembling scaffold templating the product.

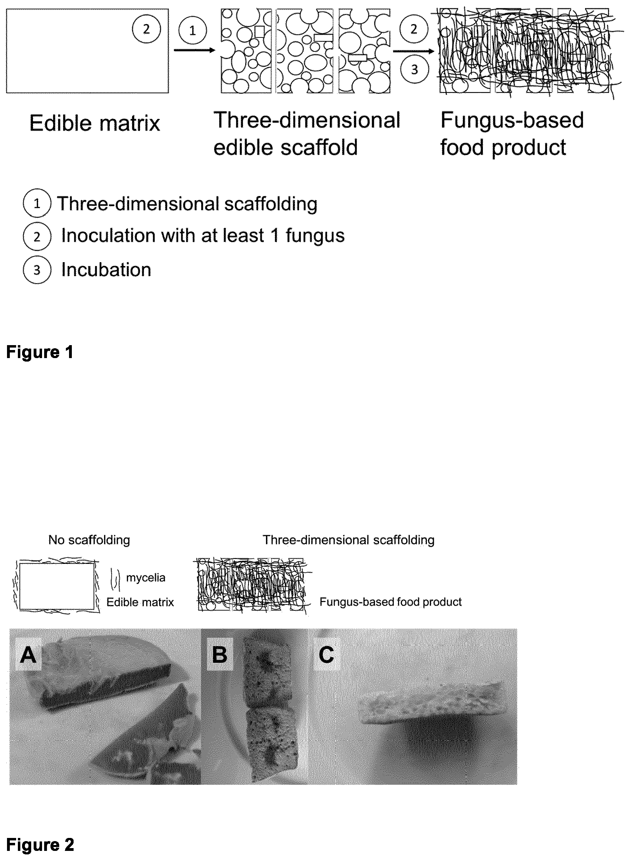

These properties can be achieved by providing a three-dimensional edible scaffold, which is inoculated with and colonized by a fungus. As will be described in further detail below, the three-dimensional edible scaffold can be designed in any desired form so as to direct the growth of the fungal mycelium, thereby being able to provide a fungal mycelium with a desired structure which may, for example, resemble the structure of an animal food product. The fungal mycelium will grow along the voids provided by the scaffold and will, together with the edible scaffold, form a coherent product with the desired texture and form. Due to the fact that the three-dimensional scaffold provides voids for the growth of the fungal mycelium, it also permits the absorption of liquids prior to or after fungal growth allowing to boost juiciness of the obtained product. The method according to the present invention is schematically depicted in Figure 1.

The term "fungus-based" means that the product obtained by the method always contains fungal components, in particular fungal mycelium, while other ingredients may vary in origin. Preferably, the product obtained by the method comprises at least 1 wt% of fungal components, more preferably at least 5 wt%, at least 10 wt%, at least 20 wt%, at least 30 wt% or at least 40 wt%, particularly preferred at least 50 wt%.

The method of the present invention is characterized in that in step (a) a three-dimensional edible scaffold is provided. The term "provided" in this context means that a preformed three-dimensional edible scaffold is used or that such a three-dimensional edible scaffold is prepared. Means and methods for preparing the three-dimensional edible scaffold are described further below.

The term "scaffold" as used in the present invention refers to a structure supporting the formation of another structure (in the present case the formation of a fungal mycelium), while said scaffold structure is made from a non-gaseous material, preferably a solid, non-liquid material, surrounding a gas-filled void space or a multitude of gas-filled void spaces. Said non-gaseous material making up the scaffold is denoted as "matrix".

The term "void" in the context of the present invention denotes an empty space surrounded by said matrix. Such a void or a plurality of voids in the scaffold structure can be formed in any possible way known to the person skilled in the art. For example, voids can be formed by stacking said matrix, by introducing a gaseous phase into said matrix, by removing liquid or solid material from said matrix, and/or by puncturing said matrix.

A void denotes any form of gas-filled space within the scaffold structure which can display any possible form as long as it allows a fungal mycelium to grow into it and/or through it. The form of the voids may depend on the way by which they are formed. For example, if the voids are formed by stacking filaments made of the edible matrix, i.e., the voids are formed at the interstices of the filaments, they will be each defined, at least partially, by the convex, outer surfaces of the individual filaments. An example of this approach is found in WO 2020/164680 A1. On the other hand, if the voids are formed by introducing a gaseours phase, e.g. bubbles, into the edible matrix (e.g. a non-solid matrix), the voids will be each defined, at least partially, by the concave, inner surface of the matrix.

Irrespective of the form of the voids, it is preferred that the scaffold comprises a multitude of voids which are (at least) partly interconnected so as to allow mycelium growth into the scaffold and through it. It is preferred that at least 50% or at least 75% of the voids, preferably at least 80%, of the voids formed by puncturing and/or at least 50% or at least 75%, preferably at least 80%, of the voids formed by foaming, form part of one or more interconnected void spaces, wherein the volume of preferably at least one of said interconnected void spaces is at least 0.05 mm³, preferably at least 0.1 mm³, even more preferably at least 0.5 mm³, even more preferably a least 1 mm³ or at least 5 mm³.

In particular, the term "void" includes "channels". The term "channel" denotes a void space connected to the outer surface of the scaffold and thus allowing penetration from outside. In a preferred embodiment a channel is understood to be an elongated tunnel-like void structure. It can have a straight form or a curved form. In one embodiment, the three-dimensional scaffold contains a multitude of channels. Such channels can penetrate the whole scaffold or can only penetrate part of the scaffold. The channels can be organized as parallel channels or their direction can be irregular. They can also cross each other and they can be interconnected. Such channels can, e.g., be created by punching with an object which leads to a channel, for example a needle, or by using a mould for the preparation of the scaffold wherein the mould shows structures which lead to channels when the matrix material is applied to the mould. Alternatively, pores can be interconnected by subjecting the foam to pressure fluctuations, or by drying, preferably under vacuum, or by microwave heating.

The term "void" further covers pores. A pore denotes a void space not necessarily connected to the outer surface. A pore can be closed and fully surrounded by the matrix or open and thus connected to other pores, and/or open to the outer surface. Preferably, the three-dimensional edible scaffold comprises open pores so that at least 50%, preferably 75%, more preferably 80% of all the pores are connected to at least one different pore. Preferably, the three-dimensional edible scaffold comprises open pores so that at least 5%, preferably at least 10%, more preferably at least 20% of all the pores are connected to the outer surface, either directly or indirectly by being connected to one or more other open pore.

The three-dimensional scaffold can display channels or pores or channels and pores or channels and/or pores in combination with any other sort of possible voids which can allow the growth of a fungal mycelium into and within the scaffold.

Pores may be closed, referred to as fully surrounded by edible matrix or open, referred to as spacially connected with another pore and/or channel or with several other pores and/or channels. Thus, the void space may be a partly or fully continuous network of pores and/or channels (possibly in combination with any other sort of possible void which can allow the growth of a fungal mycelium).

Preferably, the void space within the three-dimensional edible scaffold makes up more than 10 vol% of the three-dimensional edible scaffold, more preferably more than 20 vol%, more than 30 vol%, more than 40 vol%, more than 50 vol%, more than 60 vol%, more than 70 vol%, even more preferably more than 80 vol%. In this context, the term "void space" denotes the sum of all voids in the scaffold. When expressed as "void fraction", this denotes the volume fraction of voids in the edible scaffold. The void space is the overall volume (scaffold plus voids), measured for example by three-dimensional scanning, minus the volume of the matrix making up the scaffold, determined by weight divided by density of the material or by pycnometry.

Thus, the void space present in the three-dimensional edible scaffold may be a partly or fully continuous network of voids which may, for example, be pores and/or channels as described above. This network of voids allows the fungal mycelium to grow into and through the scaffold thereby filling the voids with fungal mycelium and consequently leading to a structure in which the voids in the three-dimensional edible scaffold are filled with fungal mycelium.

Preferably, an average size of the voids is between 20 µm to 4 cm, preferably 50 µm to 2 cm, even more preferably 100 µm to 1 cm and particularly preferably 100 µm to 0.5 cm. The term "average size of the voids" as used herein is a parameter measured by taking images, e.g., acquired by light microscopy, electron microscopy, or x-ray tomography, and measure the shortest diameter of the pore by image analysis (either in one layer or in a 3D reconstruction). Preferably, the majority of the voids (at least 50%, at least 70% or at least 90%) have a size larger than 20 µm, more preferably 50 µm. Preferably, the majority of the voids (at least 50%, at least 70% or at least 90%) have a size less than 4 cm, more preferably 2 cm.

Preferably, an average size of the voids is between 100 µm to 4 cm, preferably 500 µm to 2 cm, even more preferably 1 mm to 1 cm for three-dimensional scaffolds provided for inoculation with at least one fungus belonging to the group of basidiomycetes.

Preferably, an average size of the voids is between 20 µm to 1 cm, preferably 50 µm to 500 µm, even more preferably 100 µm to 500 µm for three-dimensional scaffolds provided for inoculation with at least one fungus belonging to the group of ascomycetes, deuteromycetes, oomycetes, or zygomycetes and no fungus belonging to the group of basidiomycetes.

It is particularly preferred that said three-dimensional edible scaffold provided according to the method of the present invention is composed of a continuous edible matrix, wherein the voids of the scaffold are introduced into the continuous edible matrix by foaming and/or puncturing.

The three-dimensional scaffold employed in the method according to the present invention is characterized in that it is edible. This means that the non-gaseous material which forms the "matrix" of the scaffold surrounding the voids is of a material which is suited for human consumption, hence palatable and non-poisonous. As regards the source of the edible material which forms the edible matrix of the scaffold, there is no particular limitation. Every edible material which can be formed into a scaffold is suitable in this context. The matrix of the scaffold can, e.g., be plant-derived, fungus-derived and/or cell-derived, in particular comprising proteins, polysaccharides, and/or lipids.

In one embodiment, the edible matrix is plant-derived. The term "plant-derived" means that at least part of the material of which the matrix is formed is derived from (a) plant(s). In this context, the term "plant" includes higher plants and lower plants, such as algae. Thus, in one preferred embodiment the matrix comprises plant material.

In one embodiment, the edible matrix is fungus-derived. The term "fungus-derived" means that at least part of the material of which the matrix is formed is derived from (a) fungus/fungi. In this context, it is preferred that the matrix is not in itself a mycelium but contains (at least in part) components which are derived from (a) fungus/fungi.

In one embodiment, the edible matrix is cell-derived. The term "cell-derived" means that at least part of the material of which the matrix is formed is derived from cultures cells, i.e. from cells which are cultivated. This covers cells from higher organisms such as plants and animals which are cultivated in vitro, e.g. in fermenters or in tissue culture. It also covers the cultivation of single-cell organisms, such as bacteria, including cyanobacteria, single-cell fungi (such as yeast) and microalgae.

In one embodiment the matrix comprises a combination of plant-derived and/or fungus-derived and/or cell-derived material.

In a preferred embodiment, the edible matrix is plant-derived and can (in addition) contain edible material derived from organisms other than plants, such as fungi or animals. However, in a preferred embodiment the edible matrix does not contain any material derived from animal slaughter or farming. In particular, the edible matrix may be vegan.

If the edible matrix is plant-derived, i.e. at least part of the material of which the matrix is formed is derived from (a) plant(s), the plant material can be any material which is suitable for the intended purpose of forming an edible scaffold. For example, it is possible to use plant parts, such as seeds, grains or fruits, preferably parts which are rich in protein, oil and/or saccharides, and to process such parts in a way which allows the plant material to be formed into a matrix. As described in the appended Examples, it is, e.g., possible to use beans or peas (of different sources), to mash them and to use the resulting mash, pulp or paste for forming an edible scaffold. However, it is of course also possible to use plant material which is already purified or partly purified, such as, e.g., plant protein or plant polysaccharide extracts, isolates or concentrates, or raw extracts from plant material in order to prepare a corresponding edible matrix.

Thus, the three-dimensional scaffold can, e.g., be made from a mash, pulp, paste, solution, suspension or emulsion, or the like, of edible material, preferably plant- and/or fungi-derived material and or cell-derived material. Such mash, pulp, paste, solution, suspension or emulsion can be filled into a mould. In particular, such mash, pulp, paste, solution, suspension or emulsion may be filled into the mould before providing it with a defined three-dimensional shape.

In particular, such mash, pulp, paste, solution, suspension or emulsion, i.e., the edible matrix, may be foamed before, after or while being filled into a mould, and the foamed or not yet foamed edible matrix yields and/or flows when being filled into the mould so that it fills the contours of said mould. It is particularly preferred, that the stress applied during the process of filling said foamed or unfoamed edible matrix into the mould overcomes the yield stress of the edible matrix. In very particular, the yield stress of said foamed or unfoamed edible matrix is below 100 Pa. Even more particular, the temperature of said foamed or unfoamed edible matrix is adjusted to avoid or decelerate solidification, in particular it is adjusted to above or below the solidification temperature of the gelling agent contained in the edible matrix.

In one embodiment the edible scaffold comprises protein. The protein can in principle be derived from any possible source, i.e. any possible organism or cell, such as animals, plants, bacteria or fungi or animal cells, plant cells or fungal cells. In a preferred embodiment, the protein is not derived from animal slaughter or farming. In another preferred embodiment the protein is derived from (a) plant(s).

The protein may be a naturally occurring protein or a recombinantly produced protein. Naturally occurring proteins are preferred. The protein may be in the form of an isolated protein which had previously been purified or partly purified from a source organism, parts thereof, tissue or from cells and/or it can be in the form of plant material which is directly processed. The protein may also be in the form of a fermented product based on plant material which contains proteins.

If the protein is derived from plants, it is preferable to use plant material which contains high quantities of protein. Examples are parts of plants, like storage organs or seeds, which are known to be rich in proteins. Suitable are in particular proteins derived from leguminous plants, such as peas, lentils, beans etc. Examples for leguminous plants from which the protein present in the edible matrix can be derived are peas (e.g. yellow peas, chickpeas), beans (e.g., soy beans, kidney beans) and lentils. Other plants from which the protein containing material may be derived are cereals (such as rice, wheat, oat, rye), corn, oilseed (such as rapeseed, sunflower seeds and pumpkin seeds) and all sorts of vegetables, such as pumpkin.

The protein can also be derived from seaweed or algae, such as Chlorella vulgaris. It is also possible to use protein from other single-cell organisms, such as yeast, e.g. as yeast extract or as yeast protein isolate or as whole yeast cells.

In one embodiment the edible scaffold contains at least one saccharide. The saccharide can in principle be derived from any possible source, i.e. any possible organism or cell, such as animals, plants, bacteria or fungi or animal cells, plant cells, fungal cells or bacterial cells. The saccharide can also be a chemically synthesized and/or non-naturally occurring saccharide. The saccharide can, for example, be selected from the group consisting of mono-, di-, oligo- and polysaccharides, e.g. complex polysaccharides, such as glucose, maltose, dextrose, mannose, alginate, alginate sulfate, gellan sulfate, starch, modified starch, gelatin, pectin, cellulose, bacterial cellulose, chitosan, agar, gellan gum, acylated and/or sulfated gellan gum, guar gum, cassia gum, konjac gum, Arabic gum, ghatti gum, locust bean gum, xanthan gum, xanthan gum sulfate, carrageenan, carrageenan sulfate, and a combination or mixture of any of the above.

In one embodiment the edible scaffold comprises protein and saccharides.

The amount of protein in the edible scaffold is not particularly limited. Preferably, the amount of protein in the edible scaffold is more than 0.1 wt%, even more preferably at least 0.5%, even more preferably more than 1%, more than 2%, more than 3%, more than 5%, more than 8%, more than 10%, more than 20% or more than 30%.

In the same manner, also the amount of saccharide, if present, in the edible scaffold is not particularly limited. Preferably, the amount of saccharide in the edible scaffold is more than 0.1 wt%, even more preferably at least 0.5%, even more preferably more than 1%, more than 2%, more than 3%, more than 5%, more than 8% or more than 10%.

In one embodiment the protein(s) and/or saccharide(s) are part of the edible matrix which is used to form the scaffold. They can be either incorporated directly into the matrix during the process of forming the matrix or they can be added later to the matrix, e.g. by absorbing a liquid comprising said protein(s) and/or saccharide(s) into the matrix.

Alternatively, the protein(s) and/or saccharide(s) can be added to the three-dimensional edible scaffold by absorbing a liquid comprising said protein(s) and/or saccharide(s) into the voids of the scaffold.

In some embodiments, the three-dimensional edible scaffold (further) comprises macro- and/or micronutrients required for fungal growth. Such macro- and/or micronutrients can be part of the edible matrix. They can, e.g., be constituted by the protein and/or saccharide present in the scaffold/matrix. They can be either incorporated directly into the matrix during the process of forming the matrix or they can be added later to the matrix, e.g. by absorbing a liquid comprising said macro- and/or micro-nutrients into the matrix.

Alternatively, the macro- and/or micro-nutrients, in particular proteins and/or saccharides can be added to the three-dimensional edible scaffold by absorbing a liquid comprising said macro- and/or micronutrients into the voids of the scaffold.

Generally, macro-nutrients required for fungal growth comprise, e.g., proteins and/or saccharides. Micro-nutrients required for fungal growth comprise, e.g., trace elements, such as organic and inorganic salts and vitamins necessary for the respective fungus or fungi.

The required nutrients may depend on the fungus/fungi used for inoculating the scaffold. Choosing the required nutrients is within the skill of a skilled person.

Advantageously, the three-dimensional edible scaffold comprises water in an amount required for fungal growth. Preferably the scaffold has a water activity of above 0.3, most preferably above 0.6. The term "water activity" denotes the vapor pressure of water above a material (partial) divided by the standard partial vapor pressure of water.

In some embodiments, the three-dimensional edible scaffold further comprises fibers, viscosifiers, fats, vitamins, trace elements, flavour compounds, colorants, spices, herbs, and/or salts.

Such fibers, viscosifiers, fats, vitamins, trace elements, flavour compounds, colorants, and/or salts can be added to the matrix separately or can be part of the material, e.g., plant material used for forming the edible matrix.

In one embodiment, the edible scaffold only contains components which cannot be consumed by the fungus which is used to inoculate the scaffold and which grows into and through the scaffold. In this case, the edible scaffold serves as a scaffold for directing fungal growth.

In another embodiment, the edible scaffold comprises components which can be consumed by the fungus while growing, e.g. which the fungus can digest and use for growth. It is preferred that the scaffold is not solely composed of components which can be consumed by the fungus but that the scaffold contains at least a certain percentage of components which cannot be consumed by the fungus and which are, therefore, maintained as a scaffold while the fungus is growing into and through the scaffold. Preferably, the percentage of components of the scaffold which cannot be consumed by the fungus is at least 1 wt%, more preferably at least 10 wt%, at least 20 wt% even more preferably at least 30 wt%, or at least 40 wt% and particularly preferred at least 50 wt%.

In a preferred embodiment, the edible matrix comprises water, agar, pea protein concentrate and/or pea protein isolate, sugar and oil or fat. The weight percentage of the respective components based on the edible matrix is, preferably, pea protein concentrate and/or isolate: 0.1-15 wt%; agar: 0.1-5 wt%; sugar: 0.01-15 wt%; and oil or fat: 0.1-10 wt%. Preferably the pH of the edible matrix is adjusted by addition of an acid or a base to a pH of between 2 and 7, preferably between 3.7 and 10, more preferably between 4 and 7. Alternatively, the edible matrix may comprise water, plant protein, at least one gelling agent, at least one nutrient source selected from the group consisting of starch, sugar and mono-/di-/oligosaccharides, and at least one ingredient that is not consumed by the fungus. The weight percentage of the respective components based on the edible matrix is, preferably, plant protein: 0.1-30 wt%; the at least one gelling agent: 0.1-5 wt%; the at least one nutrient source: 0.01-52 wt%, preferably 5-20 wt%; and the at least one ingredient not consumed by the fungus: 0.1-5 wt%. Preferably, the water activity of the scaffold is adjusted to above 0.7, more preferably above 0.8, even more preferably above 0.9. It is of particular interest that the water activity of said scaffold is adjusted to above 0.8 for scaffolds provided for inoculation with at least one fungus belonging to the group of ascomycetes, deuteromycetes, oomycetes, or zygomycetes.

The three-dimensional edible scaffold can have any form which is suitable to provide a scaffold for fungal mycelium growth. Preferably, the matrix which forms the scaffold is of a solid material. The term "solid" in the context of the present invention also comprises materials which may be considered as being semi-solid, such as a gel or a glassy or crystalline material. In some embodiments, the matrix may comprise a gel-forming polysaccharide and/or protein, wherein the gel is formed upon heating, cooling, enzymatic cross-linking and/or addition of ions. It is preferred that the edible matrix is mouldable prior to, while or after being formed into a three-dimensional edible scaffold. In particular, it is preferred to mould the edible matrix before the edible matrix is formed into a macrostructure. The macrostructure may be a structure formed by a plurality of solid or semi-solid elements having a width and/or length of more than 2 mm or more than 4 mm.

Preferably, a gelling agent that is not consumed by the fungus is used, in particular a plant-based gelling agent that is not consumed by the fungus. Such gelling agents may be agar or carrageenan.

Preferably, after solidifying, the edible matrix, as the continuous phase of the scaffold, is viscoelastic with an elastic modulus of at least 0.01 Pa, preferably at least 0.1 Pa, more preferably at least 1 Pa. The elastic modulus is determined by oscillatory rheology in the linear viscoelastic regime at a frequency of 1 rad/s.

The outer form of the scaffold is not crucial. The scaffold may, e.g., be provided in the form of a sphere, a cube, a cuboid, a cylinder, a cone, a slab, a pyramid, a slice, or any arbitrary three-dimensional shape.

The overall volume (matrix plus voids) of the scaffold which is provided in step (a) can be any volume which is suitable for obtaining the desired product. It is preferred that the overall volume is at least 1 mm³, even more preferably 1 cm³, or at least 5 cm³, or at least 10 cm³, or at least 20 cm³, or particularly preferred at least 50 cm³.

Moreover, it is preferred that the overall volume is not larger than 1 m³, even more preferably not larger than 1000 cm³.

The three-dimensional edible scaffold provided according to step (a) of the method according to the present invention can be prepared by any means and methods available to the skilled person for preparing such a scaffold.

One possibility is, for example, the foaming of an edible matrix, by dispersing gas or by dissolving gas under pressure followed by bubble nucleation. Preferably, the pressure may be later released to further expand the bubble contained in the edible matrix to thereby increase the void fraction. The term "foaming" in this context refers to a method which leads to the introduction of gas bubbles into a matrix by mechanical, chemical or physical means. Such a foaming step typically increases the volume and therefore decreases the density of a product by the introduction of air. Such a step could be used to create a three-dimensional scaffold as mentioned above by creating an empty array of channels and pores or other voids inside of the material. Preferably, the foam is solidified by gelation and/or drying. More preferably, the foam may be solidified by gelation and/or drying in a mould.

Preferably, the foaming is carried out at a temperature of above the gelation temperature of the cold-setting gelling agent or below or above the temperature at which the cross-linking enzyme causes gelation. More preferably, for an edible matrix containing agar as a gelling agent, the foaming is carried out at a temperature of above 30°C, even more preferably at above 45°C and below 95°C. In particular, for an edible matrix containing transglutaminase or laccase as cross-linking enzymes, the foaming is carried out at a temperature of not higher than 4°C.

Preferably, the step (a) of the method according to the present invention comprises steps of (a1) introducing the voids into the edible matrix, preferably by foaming; (a2) introducing the edible matrix into a mould, preferably by pouring the edible matrix into the mould; and (a3) solidifying the edible matrix in the mould, preferably by gelation. Preferably, the step (a1) is carried out before, after or at the same time with the step (a2). Preferably, the steps (a1) and (a2) are carried out before the step (a3). In other words, the matrix may be introduced into the mould before solidification (e.g. gelation). After the edible matrix has solidified to a certain extent, the matrix may be further punctured to provide more voids and/or more interconnected voids.

Preferably, the distribution of the voids in the scaffold is fixed upon solidification of the edible matrix in the mould. Preferably, the edible matrix, when initially introduced into the mould, may comprise gas/solid interfaces but does not comprise macroscopic solid/solid interfaces. The term "gas/solid interface" indicates an interface between a void and the material of the edible matrix. The term "macroscopic solid/solid interface" means an interface between two separate solid structures (e.g., of the edible matrix) in contact with each other, wherein a minimum dimension of each of the solid structures is at least 0.1 mm, preferably at least 1 mm, at least 3 mm or at least 5 mm, more preferably at least 10 mm.

It is also possible to prepare the three-dimensional scaffold by 3D printing, e.g. by printing filaments with voids in between the printing lines thereby creating a porous network containing pores and/or channels. Preferably, however, the three-dimensional edible scaffold provided by the step (a) of the method according to the present invention is not formed by this process or other similar processes involving assemblying filaments. Particularly, the step (a) of the method according to the present invention preferably does not include a step of assemblying filaments formed by extrusion. In embodiments in which the step (a1) is involved, the edible matrix has not been subjected to extrusion before being introduced into the mould. Step (a) of the method according to the present invention does not comprise an extrusion process to create multiple filaments or macrostructures. It is also possible the method of making a fungus-based food product according to the present invention does not comprise an extrusion process at all.

It is also possible to prepare the three-dimensional scaffold by using sacrificial templates in the edible matrix. Here the sacrificial templates are removed from the matrix, allowing to create a porous network. One example is using a sacrificial template that is liquefied and/or removed prior to inoculation or prior to incubation or during incubation. One example is using a thread or multiple threads in a mould and removing said threads after pouring the edible matrix into the mould and solifidying.

Another possibility for preparing the three-dimensional scaffold is the use of additive manufacturing, such as sintering-based techniques, or by freeze drying of the edible matrix. Another possibility is drying of a solution, suspension or foam, in particular microwave drying.

The three-dimensional scaffold can also be formed by puncturing a matrix with processes like puncturing, cutting, or shaping with a sharp object. Preferably, the puncture holes or channels point in one direction to create an anisotropic structure. These holes or channels may each be defined, at least partially, by a concave, inner surface of the matrix. The term "anisotropic"/"anisotropy" refers to the property of a material that allows it to change or assume different properties in different directions as opposed to isotropy. In this particular case, it refers to the growth of mycelium being "directed" into one specific direction as opposed to another direction. This has a direct effect on the mechanical properties of the obtained food product, showing fiber-like or muscle-like texture in a defined direction. In other words, the direction of mycelia fibers is predominantly in one direction as opposed to another.

In an alternative method, the three-dimensional scaffold can also be formed by foaming followed by puncturing to create porosity or pores and channels of different size and shape.

The combination of several of the above-described methods or techniques allows to create primary and secondary porosity or voids with varied sizes and shapes.

In a preferred embodiment, the three-dimensional edible scaffold is composed of a continuous edible matrix, wherein voids in the scaffold are introduced into the continuous edible matrix, e.g. by foaming and/or puncturing. The term "continuous" denotes, in the scope of the present invention, that a material, including voids or void space, forms an uninterrupted whole in space, where each partial volume of a material is in contact with at least two neighboring volumina except at the surface layer. A continuous material, which is interconnected at said contact points through physical and/or chemical bonds and/or steric hindrance is denoted as "coherent". Thus, in this embodiment, the three-dimensional edible scaffold is formed by first providing a continuous edible matrix which is then subsequently converted into a three-dimensional scaffold by introducing voids into the matrix. Preferably the voids are introduced into the matrix by foaming or puncturing or a combination thereof. The edible matrix may be continuous when initially introduced into the mould.

In one embodiment, said three-dimensional edible scaffold is a dried scaffold which is re-wetted/rehydrated prior to inoculation with the fungus according to step (b) of the method according to the invention. In particular, the scaffold can be a scaffold which is dried to a water activity of below 0.6, preferably below 0.3 and re-wetted to a water activity required for growth of the respective fungi. The term "re-wetting/rehydration" refers to the addition of water to a material through capillary suction and/or swelling. Rehydration should increase the water activity to the level required for fungal growth.

Preferably, re-wetting/rehydration is performed by soaking in water and/or spraying water onto the outer surface of the three-dimensional edible scaffold or by increasing the relative humidity of the surrounding gas phase. This water may be enriched with nutrients for fungal growth or human consumption or with flavour compounds, colouring compounds, salts, acids and/or bases. In this way a three-dimensional edible scaffold can be provided comprising nutrients for fungal growth and optionally nutrients for human consumption.

Thus, in this embodiment the step of providing a scaffold according to step (a) of the method comprises the providing of a dried three-dimensional edible scaffold and of wetting the scaffold prior to the inoculation step (b) of the method.

According to step (b) of the method according to the present invention, the three-dimensional edible scaffold is inoculated with at least one fungus so as to generate an inoculated scaffold.

The inoculation can be effected by means and methods known to the person skilled in the art. In particular, the inoculation can be achieved through contact of the fungus or fungi with one single surface, multiple surfaces or all surfaces of the three-dimensional edible scaffold. The term "surface" here means outer surfaces as well as "inner surfaces" which represent sufaces of voids in the scaffold. Optionally, the term "outer surface" when used in association with an entity means a surface that substantially envelopes said entity or surfaces that, when considered collectively, substantially envelope said entity, while the term "inner surface" when used in association with an entity means a surface or surfaces located substantially within that enveloping surface.

Generally, inoculation can be achieved by applying any form of the at least one fungus to the scaffold which is capable of growing into a mycelium. In one embodiment, inoculation is achieved by applying mycelium and/or spores of the at least fungus to at least one surface of the scaffold. If mycelium is used, the mycelium is preferably applied in wet form, e.g. in water or in another suitable liquid. Spores can be applied, e.g., as dried spores or as spores dispersed in a liquid phase, for example in water. When using spores for inoculation, the inoculation can also be achieved by mechanically introducing the spores into the scaffold, e.g. by pressing them into the matrix of the scaffold.

If more than one fungus is used for inoculation, the fungi can be added at the same time or at different times.

The inoculation can be performed after the three-dimensional scaffold has been formed. However, it is also possible to effect inoculation of the scaffold while creating the scaffold. This can be achieved, e.g. by already inoculating the edible matrix which is used to form the scaffold or by adding the at least one fungus during the formation step of the scaffold, e.g. during a foaming or puncturing process. In this context, it is possible to use spores and/or mycelia so as to incorporate them into the matrix so that the matrix already contains the spores and/or mycelia and is, thereby, inoculated with the fungus.

Thus, steps (a) and (b) of the method according to the present invention can also be carried out in one step.

It is also possible to add the at least one fungus prior to or after the foaming and/or puncturing process. Most preferably, the at least one fungus is added to the edible matrix prior, while or after foaming, but prior to puncturing.

The inoculation can also be carried out as a directed inoculation. This term means that inoculation is only carried out in partial regions of the three-dimensional scaffold, in particular at a plane of the outer volume, preferably at the top plane, or at the bottom plane. Thus, fungal mycelia grow starting from the inoculated plane into the free void space of the scaffold resulting in the formation of a directed mycelium structure.

The fungus used in the method according to the invention for inoculating the edible scaffold is not particularly limited as long as it is capable of growing a fungal mycelium and as long as it is edible. The term "fungal mycelium" denotes the vegetative part of a fungus consisting of a mass of branching, thread-like hyphae.

In particular, any mushroom can be used which provides for a filamentous body structure. Thus, it is possible to use any filamentous fungus from any of the divisions of the phylum fungi. In one embodiment, the at least one fungus is a filamentous fungus selected from the group consisting of ascomycetes, basidiomycetes, deuteromycetes, oomycetes, and zygomycetes. If more than one fungus is used, it is possible to use fungi from the same type or from different types as listed above.

Preferably, the at least one fungus is selected from the group consisiting of Zygomycota and Ascomycota, preferably from a genus selected from the group consisting of *Aspergillus, Rhizopus, Penicillum* and *Fusarium.* Even more preferably, the at least one fungus belongs to a species selected from the group consisting of *Aspergillus oryzae, Rhizopus oligosporus, Penicillum camemberti* and *Fusarium venenatum.*

In another preferred embodiment, said at least one fungus is selected from the division of basidiomycetes, preferably from a genus selected from the group consisting of *Pleurotus, Laetiporus, Lentinula, Ganoderma, Grifola, Agaricus, Flammulina, Morchella, Hypholoma, Macrolepiota* and *Cantharellus.* Even more preferably, the at least fungus belongs to a species selected from the group consisting of *Pleurotus sapidus, Pleurotus ostreatus, Pleurotus djamor, Laetiporus sulphureus, Lentinula edodes, Ganoderma lucidum, Grifola frondosa, Agaricus bisporus, Flammulina velutipes, Morchella angusticeps, Hypholoma capnoides, Macrolepiota procera* and *Cantharellus cibarius.*

In another preferred embodiment the at least fungus belongs to the class of Agaricomycetes.

After the inoculation step (b) of the method according to the invention, the inoculated scaffold is incubated at growth conditions that allow for mycelium growth of the at least one fungus so that the at least one fungus grows through the scaffold as a mycelium to form a fungus-based food product.

This incubation step may directly follow the inoculation step. Alternatively, if it is not intended that mycelium growth starts immediately, the inoculated scaffold may also be treated or subjected to conditions which prevent mycelium growth. By such a measure, the inoculated scaffold can be stored until the incubation step starts. It is, for example, possible that the inoculated scaffold is dried to below the water activity required for fungal growth and stored at the respective conditions prior to incubation for mycelium growth. It is also possible that the inoculated scaffold is stored at temperatures below the temperature required for fungal growth prior to incubation for mycelium growth.

The incubation according to step (c) of the method according to the present invention is performed under conditions which allow for the growth of the mycelium of the fungus/fungi used for the inoculation according to step (b). The growth conditions depend on the fungus/fungi and can be adapted by the skilled person accordingly. The term "allow for mycelium growth" means that the conditions allow for the growth of mycelium but do not allow for the formation of and/or differentiation into fruit bodies. Thus, under the conditions used for incubation only mycelium growth occurs but no formation of and/or differentiation into fruit bodies.

The incubation allows the mycelium to grow along the surfaces of the scaffold, in particular also into the voids of the scaffold, thereby filling up the voids in the scaffold with mycelium.

Preferably, the incubation is carried out for at least 6 hours, more preferably at least 12 hours, even more preferably at least 24 hours.

In an alternative embodiment, the inoculated scaffold is sheared or cut or flushed with air or oxygen-containing gas or expanded by application of vacuum after a certain duration of incubation, preferably when the oxygen concentration has decreased by more than 10%, more preferably by more than 50%, in particular the inoculated scaffold is sheared after at least 30 minutes of incubation, more preferably after 1 hour of incubation. Upon shearing or cutting, the inoculated scaffold is disintegrated into pieces or crumbles of at least 1 mm in width and/or length, preferably at least 2 mm, more preferably at least 5 mm in width and/or length.

Preferably, more oxygen and/or nutrients, such as a nitrogen-containing material, preferably a protein, and/or a saccharide is added to the sheared or cut incubated scaffold.

The pieces or crumbles may be filled into another mould for incubation before or after adding additional oxygen and/or nutrients. Additional gelling agent may be added to the pieces or crumbles prior to filling them into said mould, whereas said gelling agent may be a gelling polysaccharide, a gelling protein or only a cross-linking agent, such as a cross-linking enzyme or an ion, preferably cation. In particular, the inoculated, incubated crumbles may be mixed with more inoculated scaffold or edible matrix prior to solidification.

Depending on the edible matrix chosen for the formation of the scaffold, it is also possible that the mycelium grows into the edible matrix of the three-dimensional edible scaffold and penetrates the matrix. It is also possible that the mycelium grows into the matrix and consumes the matrix or part of it. This is in particular the case if the edible matrix contains macro- and/or micro-nutrients required for fungal growth. In this embodiment, the growth of the fungus mycelium is preferably controlled so as to make sure that the matrix is not completely consumed by the fungus during mycelium growth but that at least a part of the matrix which builds the edible scaffold is maintained. Preferably at least 5 wt% of the matrix, more preferably at least 10 or 20 wt%, even more preferably at least 30 wt%, or at least 40 wt% and particularly preferred at least 50 wt% of the edible matrix which builds the scaffold are maintained and not consumed by the fungus during growth.

Inasfar as the edible matrix which forms the scaffold contains components which are not required for fungal growth and which are not or cannot be consumed by the fungus during growth, these components remain in the resulting fungus-based food product and contribute to the texture and nutritional value of the food product. Thus, the product texture can be fine-tuned by tailoring the mechanical properties of the non-digested scaffold.

It is preferred that the incubation step (c) of the method according to the present invention is carried out for a time and/or under conditions which are sufficient to achieve a growth of the mycelium so that it fills the void space in the three-dimensional edible scaffold completely, preferably that each void contains mycelium. However, it is also possible to stop the step of incubation and/or the fungal growth, if desired, at an earlier point in time when the fungal mycelium did not yet fill the void space completely. However, it is preferred that the fungal mycelium at the end of the incubation step (c) is contained in at least 10% of the voids, more preferably at least 20%, even more preferably at least 30%, at least 40%, at least 50%, at least 60%, at least 70% or at least 80% and particularly preferred at least 90% of the voids.

The term "filled" with regard to a void space filled with fungal mycelium preferably means that at least 2 vol% of the void space is occupied by fungal mycelium, more preferably that at least 5 vol%, more preferably at least 10 vol%, most preferably at least 20 vol% of the void space is occupied by fungal mycelium.

During the incubation step, fungal mycelium growth can be controlled by changing the environmental growth conditions, including time, humidity, and temperature, which affect mycelium density, morphology, and growth. In any event, the applied environmental growth conditions are chosen in a manner that they permit growth of mycelium without formation of/differentiation into fruit bodies.

As most fungi require oxygen, the fungal mycelium grows into the voids along the surface of the scaffold and fills up the voids. In a preferred embodiment, it is ensured that the diameter of the voids in the scaffold is not larger than double the growth height of the fungus in free space. In this context, the term "growth height" denotes the height measured from a surface, which is achieved when fungal mycelium grows on a nutrient-containing surface into free space. At larger diameters, the void may not completely be filled with fungal mycelium leading to a remaining void space in the fungus-based food product. As the fungal mycelium grows through the whole scaffold along the surface, and, depending on the fungus species chosen for inoculation and/or the composition of the edible matrix, also into the matrix, the initial shape and size of the scaffold templates the size and shape of the fungus-based food. The size, shape and orientation of the fungal mycelium in the fungus-based food product is in turn templated by the void space in the scaffold. Thus, a scaffold with long channels allows to produce long fungal mycelium fibers resembling the structure of muscle fibers in meat. Additionally, fungal growth speed, morphology, and mycelia thickness can be fine-tuned by changing the temperature and general growth conditions such as humidity and oxygen/carbon dioxide supplies accordingly. The fungi ferment the products surrounding them by using a variety of enzymes, contributing to the taste of the product. Using one or several fungi in combination, the taste can be tailored to suit consumer preferences without the need of adding flavours.

As the fungal mycelium grows into the voids of the scaffold, a coherent food product (if desired with meat-like texture) is formed. Additionally, the voids provided by the scaffold permit absorption of liquids prior to or after fungal growth allowing to boost juiciness. The texture of the product can further be controlled by fine-tuning the formulation and thus the mechanical properties of the scaffold. If desired, the voids allow to create a global fiber-like texture across the whole food product. Growth of mycelium across the entire food matrix can be directed by directed inoculation, nutrient gradients or by introducing directed/anisotropic voids such as parallel channels, in turn creating directionality and, thereby, meat-like fibers. Thus, it is, e.g., possible to achieve a directed growth of the mycelium by anisotropic voids in the three-dimensional edible scaffold and/or by providing a path for mycelia growth through defined channels of the three-dimensional scaffold, or by providing a growth path for mycelia through nutrient and oxygen gradients or by directing the mycelial growth by inoculating fungi only in partial regions of the three-dimensional edible scaffold.

With this directionality, mycelium growth can be directed into a specific direction, resembling the directionality observed in meat products.

In a preferred embodiment, the edible scaffold of the product comprises channels which are filled during incubation by fungal mycelium thereby forming fiber-like fungal structures with an aspect ratio of above 1. In this context, "aspect ratio" denotes the ratio of length over diameter of the grown mycelium structure templated by the void space.

The method according to the present invention may further comprise the step (d) of interrupting the growth of the at least one fungus. This can be achieved by any means and methods known to the skilled person to be suitable to stop growth of the corresponding fungus/fungi which had been chosen for inoculation. Preferably, the interruption of fungal growth is achieved by changing the temperature and/or water activity to below or above the temperature and water activity conditions required for growth of the at least one fungus. Alternatively, growth is interrupted as micro- and/or macronutrients are depleted. In this case, the end of fungal growth is predetermined by the provision of the nutrients provided. It is also possible to interrupt fungal growth by subjecting the product obtained in the incubation step (c) to a step of exposing it to a liquid which is absorbed by the product (as described further below).

After the incubation step and after fungal growth stopped, it is also possible to subject the obtained product which consists of the scaffold which is filled with mycelium to a washing step. This washing step may serve to remove, e.g. residual nutrients or loose edible matrix parts. This washing step can be done with any suitable solution, e.g. with water, oil and/or organic solvents.

The method according to the present invention may further comprise the step of exposing the obtained fungus-based food product to a liquid so that the food-product absorbs the liquid, e.g. into the remaining void space which is not filled by fungal mycelium by capillary absorption or into the edible matrix by swelling. This step can be carried out after the incubation step (thereby preferably leading to the interruption of fungal growth) or after the interruption step (d). Any liquid which is suitable to be absorbed by the obtained mycelium-filled scaffold can be used as long as it is edible. In some embodiments, the absorbed liquid is water-based and/or oil-based. In a preferred embodiment, the liquid is further enriched with flavour compounds, colorants, viscosifiers, fibers, vitamins, trace, elements, salts, polysaccharides, and/or proteins.

Such an absorption of a liquid can be used to increase the juiciness of the food product, which is often lacking in meat alternatives. In this context, the term "juiciness" denotes a sensory attribute perceived when eating e.g., fresh fruit and vegetable (water release) or meat (water and oil release), and describes the amount of liquid released during mastication, the force at which the juice is expelled, the amount of juice released at the first bite and over time, the consistency of the juice, and the contrast between liquid and solid. Thus, juiciness requires that a food product can hold a liquid and release the liquid upon compression or breaking such as during mastication.

The method according to the present invention may furthermore comprise the step that the obtained fungus-based food product is cut, pulled, and/or pressed into pieces or slices to mimic the shape of meat products.

In another aspect, the present invention also relates to a fungus-based food product obtainable or obtained by the above-described method of the present invention.

As decribed above, the method according to the present invention allows to prepare a fungus-based food product comprising a three-dimensional edible scaffold comprising an edible solid matrix, wherein the voids in the three-dimensional edible scaffold is filled with fungal mycelium.

In a preferred embodiment, the fungus-based food product contains at least 10 wt% edible scaffold, more preferably at least 20 wt% edible scaffold, even more preferably at least 30 wt% edible scaffold, or at least 40 wt% edible scaffold, particularly preferred at least 50 wt% edible scaffold.

The term "wherein the voids in the three-dimensional edible scaffold is filled with fungal mycelium" means that the mycelium fills up at least part of the volume created by the voids in the scaffold. It is preferred that the fungal mycelium fills up at least 2 vol% of the void space, more preferably at least 5 vol%, even more preferably at least 10 vol%, at least 20 vol%, at least 30 vol% or at least 40 vol%, particularly preferred at least 50 vol%. In an especially preferred embodiment, the mycelium is contained in 100% of the voids.

In certain embodiments, not the whole void space is filled with fungal mycelium. In these embodiments, it is preferred that at least part of the void space is filled with liquid. As regards the liquid which can be introduced into the food product, the same applies as has been set forth above in connection with the method of the present invention.

In general, as regards the composition and structure of the three-dimensional scaffold and/or matrix and the preferred embodiments, the same which has already been described above in connection with the method of the present invention also applies in connection with the product.

Thus, in particular as regards the composition of the matrix, which forms the backbone of the scaffold, the same applies as has been described above in connection with the method of the present invention.

Also, as regards the fungus used for inoculation and the further treatments of the scaffold prior to inoculation, after inoculation or after incubation, the preferred embodiments described above in connection with the method of the present invention also apply to the obtained product.

As described above, in connection with the method of the present invention, the structure formed by the fungal mycelium and present in the fungus-based food product is templated by the size and shape of the voids of the three-dimensional edible scaffold. In a preferred embodiment, the edible scaffold of the product comprises channels which are filled by fungal mycelium thereby forming fiber-like fungal structures with an aspect ratio of above 1.

In another preferred embodiment, the diameter of the voids in the scaffold is smaller than double the growing height into free space of the respective fungus comprised in the product.

In some embodiments, the formed mycelium structure is anisotropic.

In some embodiments, the edible solid matrix present in the fungus-based food product comprises more than 0.1 wt% protein or saccharide. As regards the definition of proteins and saccharides, the same applies as has been set forth above in connection with the description of the scaffold and matrix in the context of the method of the present invention.

In some embodiments, the fungus-based food product comprises fibers, viscosifiers, fats, vitamins, trace elements, flavour compounds, colorants, acids, bases, spices, herbs, and/or salts.

The fungus-based food product of the invention comprises a three-dimensional edible scaffold made of an edible matrix with voids, wherein the voids in the three-dimensional edible scaffold are filled (completely or partly) with fungal mycelium. The edible matrix or the food product can contain residues of micro- and/or macronutrients required for fungal growth, water, and, optionally, further components contributing to texture, nutritional value, taste, colour and shelf-life of the food product. The method for producing the fungus-based food product can be adjusted (as described above) so that mycelia grown through the void space of the scaffold and, optionally, through the edible matrix forming the scaffold are in the same length scale as muscle fibers. Thus, such mycelia are able to mimic the structure of meat. Such as product has i) directed or controlled fiber-like mycelium structure/network spanning across the entire structure templated by the void space, ii) a global meat-like texture created by the mycelium structure/network in combination with the tuned edible scaffold, and iii) juiciness created through the porous matrix. The texture of the fungus-based food product can be tuned by the size, shape and fraction of the fungal mycelium, choice of fungi, and growth conditions applied for the respective fungus or fungi combination, as well as by the mechanical properties of the edible matrix making of the scaffold and by the scaffold meso-/micro-structure itself. For the latter two, e.g. the fraction of proteins and saccharides and other edible components, the network-forming ability of the used components, the physical and chemical cross-links formed in between these components as well the shape of voids and struts, among other aspects, can be used to modify product texture. Based on the method of the present invention, a fungus-based food product can be provided which resembles animal meat products and can be handled accordingly, such as by frying in a pan with minor change in shape and water content. The juiciness of such a product may be further increased by soaking the fungus-based food product in water- and/or oil-based liquid prior to cooking/frying/heating and consumption. Furthermore, the colour and taste of the fungus-based product can not only be tuned by addition of flavour compounds and colourants but alternatively by choice of fungi. Different fungi may result in differently coloured products, not only through mycelium growth but also through sporulation. The fungi may further form flavour compounds through enzyme production, which in turn transform the surrounding organic matter, preferably proteins, into flavours.

In a preferred embodiment, the fungus-based product is heated, pasteurized, sterilized and/or smoked prior to further processing or consumption. Preferably, the fungus-based product is heated or cooled or frozen to inactivate the fungus.

The terms **"comprising", "comprises"** and **"comprised of"** as used herein are synonymous with **"including"** or **"includes";** or **"containing"** or **"contains",** and are inclusive or open-ended and do not exclude additional, non-recited members, elements or steps. The terms **"comprising", "comprises"** and **"comprised of"** also include the term **"consisting of".**

As used herein the term **"about"** means approximately, in the region of, roughly, or around. When the term "about" is used in conjunction with a numerical value or range, it modifies that value or range by extending the boundaries above and below the numerical value(s) set forth. In general, the term "about" is used herein to modify (a) numerical value(s) above and below the stated value(s) by 10%.

### Brief description of Figures

The invention is further illustrated by the following examples and figures, from which further embodiments and advantages can be drawn. These examples are meant to illustrate the invention but not to limit its scope.
**Figure 1****:** Description of scaffolding process. The chosen ingredients are shaped into an edible matrix (which may optionally already be inoculated with fungus). This matrix is scaffolded into a porous scaffold that has voids, e.g., made up of pores and/or channels. The scaffold is inoculated with at least one fungus (in particular if the matrix had not already been inoculated with a fungus). Fungus growth is triggered by environmental growth conditions referred to as incubation. After incubation, the fungus-based food product is covered internally and externally by fungal mycelium.
**Figure 2****:** Comparison of mycelium growth on a matrix without voids and scaffold made of a matrix with voids. (A) A matrix made of agar, malt extract, and yeast extract without voids, hence not made into a scaffold, was inoculated with *Aspergillus oryzae* at the outer surface and incubated at 32°C and 80% relative humidity for 48 hours. The fungal mycelia grew mainly on the outer surface of the matrix and had only little penetration into the matrix. (B) A matrix made of pea protein, agar, and malt extract was inoculated and incubated with *Aspergillus oryzae* at the same conditions as in (A). Fungal mycelium filled up the void space during incubation. Growth of (A) and (B) was terminated by removing the scaffold from incubation conditions. (C) Scaffold consisting of chickpeas, yellow peas, agar and malt extract was inoculated with the same fungus and incubated at same conditions as in (A) and (B). As in (B) the fungal mycelium grew into the void space of the scaffold. Schematics above illustrate the difference in mycelium growth with and without voids.

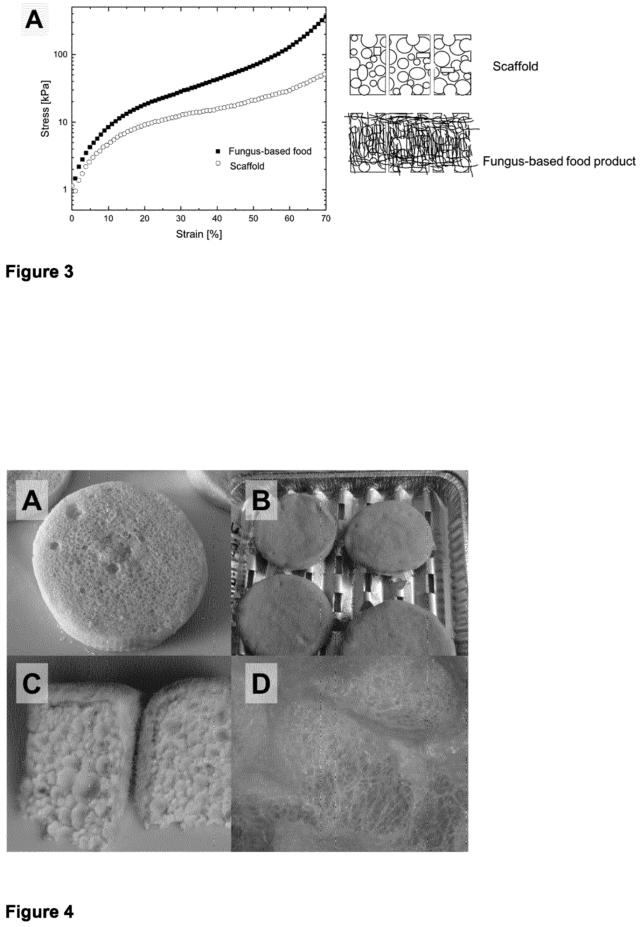

**Figure 3****:** Mechanical response in uniaxial compression of a three-dimensional edible scaffold made from pea protein and agar by foaming and gelation without fungi compared to the same scaffold inoculated with *Aspergillus oryzae* and incubated at 32°C and 80% relative humidity for 48 hours resulting in a fungus-based food product. The growth of fungal mycelium into the voids of the scaffold led to an increase in stiffness and strength, perceived as an alteration in texture.
**Figure 4****:** A chickpea-based scaffold (A) is inoculated with *Aspergillus oryzae* and incubated for fungal growth at 30°C and 80% relative humidity for 48 hours (B). Fungal growth occurred through the entire structure, including surface and interior of the scaffold (C). A close-up of the pores reveals that the mycelia grew into the entire void space of the scaffold (D).

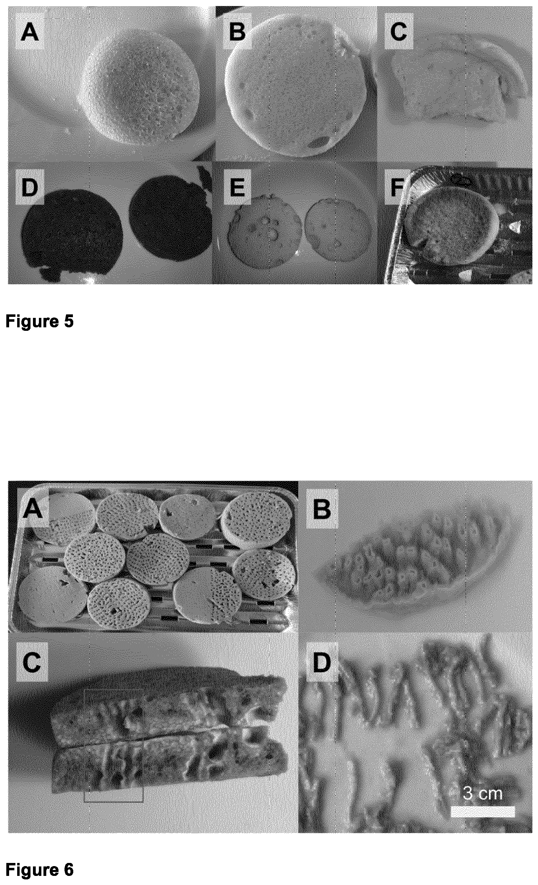

**Figure 5****:** Based on the the presented approach, a variety of plant-based foams acting as scaffolds were inoculated and incubated to make fungus-based food products. Water-based foam made of (A) 2 wt% agar and 10 wt% maltose sugar, (B) 2 wt% agar with Cremodan (commercial surfactant based on fatty acids), (C) 10 wt% soy protein isolate with 2 wt% agar and 10 wt% maltose (D) 10% kidney beans and 2 wt% agar with 10 wt% maltose, (E) 10 wt% pea protein isolate with 2 wt% agar and 1 wt% maltose, (F) 10 wt% oat protein with 2 wt% agar and 2 wt% maltose. All foams were inoculated with *Aspergillus oryzae* and incubated at 32°C and 75% relative humidity over 48 hours.
**Figure 6****:** Alternative scaffolding approach. Scaffolding was achieved by mechanically introducing channels into an edible matrix. (A) The edible matrix can be perforated (or partially perforated) by punching through channels mechanically or by making an edible matrix in a perforated fashion by moulding (B). As the fungal mycelium grows into the long, thin channels, meat-like fibers are formed. By removing the scaffold material (B, D), it is possible to isolate and visualize the single fiber structures shown in (C). The resulting mycelium-based fibers mimic the muscle fibers of meat.

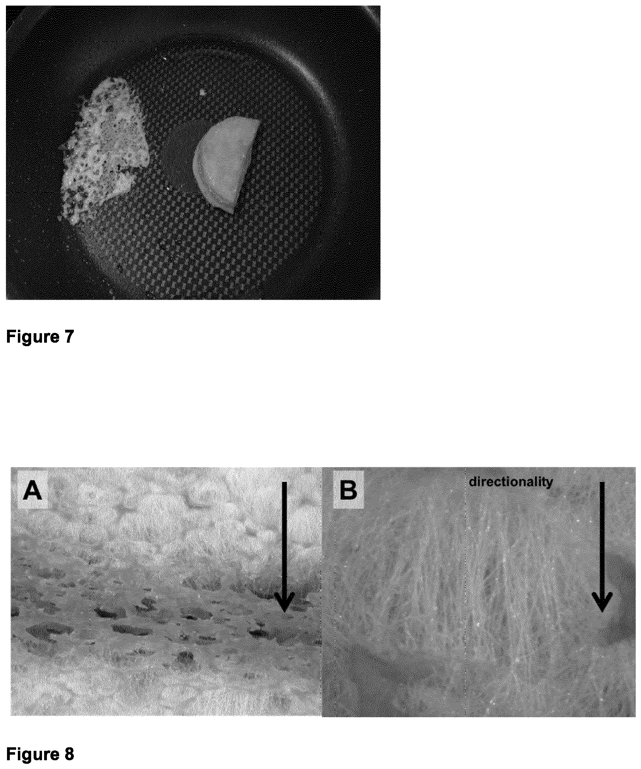

**Figure 7****:** Frying and cooking stability of over-grown fungus-based food products. The stability of a fungus-based food product grown with mycelium through the material while frying in rapeseed oil at high temperatures (>100°C) is shown. On the left side of the pan, the edible three-dimensional scaffold without any fungal growth is fried, while on the right side, the fungus-based food product made from the same scaffold is fried. As visible, the fungus-based food product stays stable as the mycelium forms a coherent network throughout the product, while the scaffold without fungal mycelium loses its structure and collapses.
**Figure 8****:** Directionality of mycelia in fungus-based food product. (A) demonstrates the three-dimensional edible scaffold with directed inoculation from the top plane, hence fungal mycelium grows from the top plane downward in one direction. (B) Close-up of a single pore with mycelia growing in one controlled and defined direction.

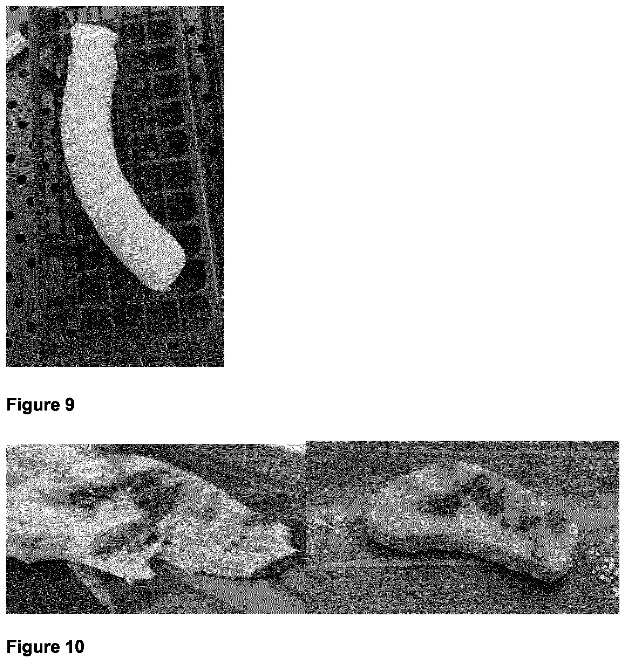

**Figure 9****:** A myceliated scaffold moulded in a sausage-shaped mould and incubated to allow mycelial growth through and on top of the three-dimensional scaffold.
**Figure 10****:** Photograph of a fungus-based food product myceliated by A. oryzae and pan fried in oil.

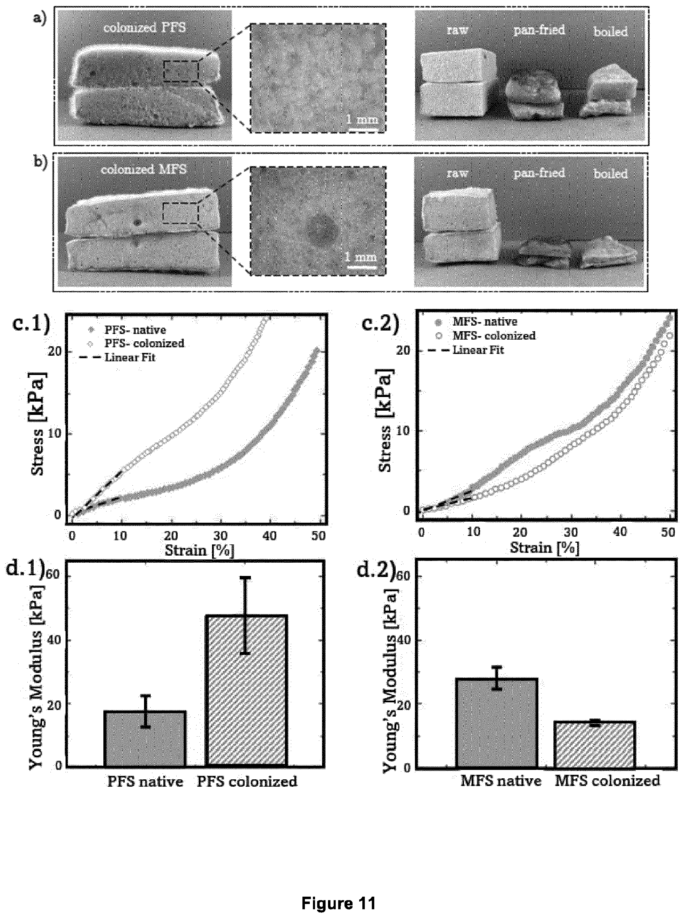

**Figure 11****:** Comparison of two different foaming techniques according to Example 31, as images in a) and uniaxial compression tests in b) and c).

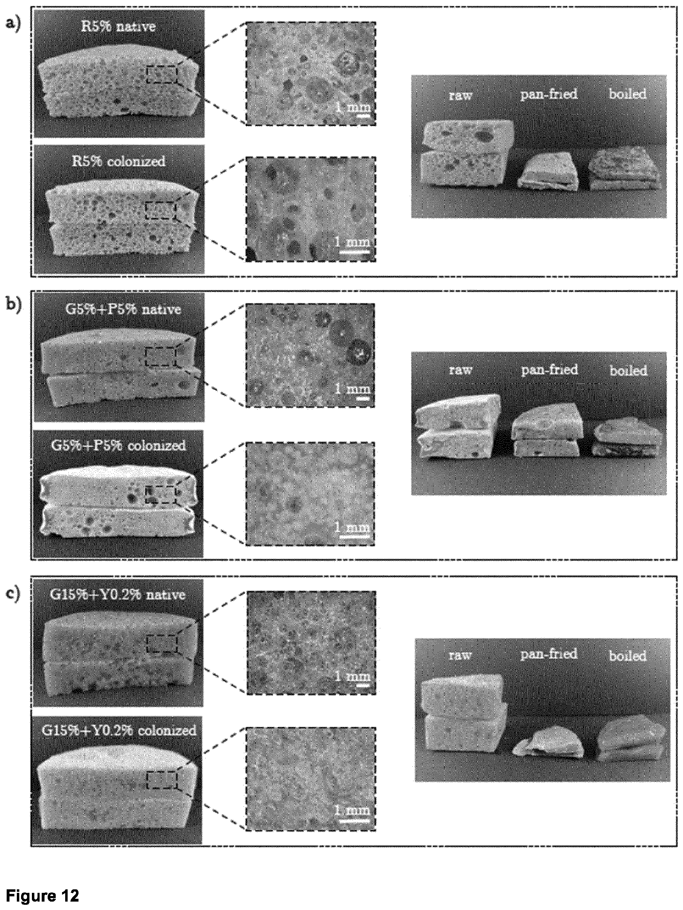

**Figure 12****:** Comparison of three different formulation according to Example 32 with different nitrogen sources, being rice protein in a), pea protein concentrate in b), and yeast extract in c) and with and without sugar.

### Examples

### Example 1

Figure 2 shows the comparison of fungal mycelium growth on a matrix without voids (A) compared to a scaffolded matrix with voids (B, C). For the preparation of the matrix without voids, 1.5 wt% agar, 10 wt% malt extract and 3 wt% yeast extract were dispersed in water followed by cooking and moulding. Upon cooling, the agar gelled resulting in a solid matrix without voids. This gelled matrix was inoculated at the outer surface with *Aspergillus oryzae* spores dispersed in water. After incubation at 32°C and 80% relative humidity over 48h, the fungal mycelium had only covered the surface of the matrix and only minorly penetrated into the matrix. The scaffolded matrices were made by dispersing (B) 10 wt% pea protein, 1.5 wt% agar and 10 wt% malt extract, and (C) 10 wt% mashed yellow peas and chickpeas with 1.5 wt% agar and 10 wt% malt extract in water followed by cooking, foaming in a cream whipping device, moulding, and gel-setting. Upon gelation a stable scaffold with pores and channels was formed, which was inoculated with spores of *A. oryzae* in water followed by incubation at same conditions as (A). In contrast to (A), the mycelium grew in the voids of the scaffolds and filled the whole scaffold. The fibrosity of this product was given directly by the porosity and the mycelium growth.

### Example 2

An edible three-dimensional scaffold was formed by foaming a solution of 10 wt% mashed beans, 10% maltose, 2 wt% agar. The three-dimensional edible scaffold was inoculated with dry spores of *Aspergillus oryzae* from every side and incubated for 48 hours at 32°C and 75% relative humidity. To demonstrate how growth through the matrix affects the mechanical properties of the resulting fungi-based food product, compression tests were performed using a mechanical testing device (Figure 3). The mechanical response of a cylindrical sample (2.5 cm in diameter, 2 cm in heights) in uniaxial compression at 2 mm/s showed a higher stiffness, higher energy absorption capacity and higher hardness for the obtained fungus-based food product compared to the corresponding scaffold which had not been inoculated with the fungus due to an interlinkage of the three-dimensional scaffold by mycelium. Through mycelia growth, the edible matrix is interconnected, thus resulting in enhanced mechanical stability, which in turn correlates to the perceived texture.

### Example 3

An edible matrix with 30% mashed whole chickpeas and water was mixed with 2 wt% agar and with citric acid to reach a pH value of 4.5 and heated to 95°C. This hot slurry was then introduced into a cream whipping device and pressurized with N₂O to reach a pressure of 15 bar. The device was agitated over 10 min to partly disperse and partly dissolve the gas in the slurry. The slurry was released from the device leading to an expansion of the gas and hence foaming of the slurry. Upon cooling, the foamed slurry gelled due to the contained agar. With this technique, a void fraction of about 50% was reached. The resulting porosity was further increased by mechanically introducing pores with a sharp object. The resulting porous scaffold was then inoculated with spores of *Aspergillus oryzae.* The foam was inoculated by slight compression of the scaffold in a water solution with fungal spores. After compression release, the water uptake into the scaffold led to an evenly distributed inoculation with fungi. The inoculated foam was then incubated at 32°C under high ambient humidity of 80%. The resulting foam structure after 40h was then fried (>100°C) in oil to prevent further fungal growth. The resulting fungus-based food product has mycelia growth throughout the entire material thanks to the open porous structure across the entire fungus-based food product (Figure 4).

### Example 4

Three-dimensional scaffolds were created from various plant-based matrices. The compositions were as follows: (A) 2 wt% agar and 10 wt% maltose sugar, (B) 2 wt% agar with Cremodan^{®} (commercial surfactant based on fatty acids), (C) 10 wt% soya protein isolate with 2 wt% agar and 10 wt% maltose (D) 10% kidney beans and 2 wt% agar with 10 wt% maltose, (E) 10 wt% pea protein isolate with 2wt% agar and 1 wt% maltose, (F) 10 wt% oat protein with 2 wt% agar and 2 wt% maltose. The mixtures were heated to 95°C, introduced into a vessel and pressurized with N₂O to reach a pressure of 15 bar. The slurries were mixed with the gas for around 10 minutes. The slurries were released from the vessel leading to an expansion of the gas, hence foaming of the slurries to reach void fractions of about 30 vol%-70 vol%. The resulting porosity was further increased by mechanically introducing pores with a sharp object. Inoculation with *Aspergillus oryzae* of these foams was done by a variety of techniques ranging from inoculation with alive mycelia in water, spores in water, and dry spores. All inoculated scaffolds were incubated at 30-35°C at relative humidities of 65-80% over 40-48 hours. As shown in Figure 5, the final products obtained after incubation are entirely overgrown with fungal mycelium to form a fungus-based food product.

### Example 5

A scaffold was created by first producing an edible matrix composed of proteins and polysaccharides and then subsequently adding voids/pores to the matrix by mechanically punching through the matrix. An edible matrix with 2wt% agar and 40% mashed chickpea beans was heated to 90°C and cooled down to room temperature. The resulting hardened agar was made porous by introducing channels through the puncturing with a sharp needle with a diameter of 2 mm. This was done selectively and randomized (Figure 6 A). The scaffold was inoculated with spores from *Aspergillus oryzae* at 30°C, 80% relative humidity for 40 hours. After growth, the directed mycelia growth was observed in the direction of the channels. These channels enabled the formation of long fibers corresponding to the exact length of the punctured channels. The thickness of these channels directly corresponds to the thickness of the formed fibers. By solubilizing the three-dimensional scaffold, a meat-like fiber structure appears that reveals fibers of fungal mycelia that are aligned in a specific direction (Figure 6 B, C, D). The same result was obtained by using a mould with spikes that allowed to form a porous scaffold immediately.

### Example 6

A three-dimensional scaffold with mycelia and without mycelia were compared in a frying test (Figure 7). While the fungus-based food product (right) showed browning upon frying, remained stable and did not lose more than 20% of its volume, the same three-dimensional scaffold without fungus lost its shape and melted immediately upon frying. This demonstrated that the final fungus-based food product is heat stable and can be used for cooking purposes showing similar frying behavior as a piece of meat.

### Example 7

A three-dimensional porous scaffold was created by 3 wt% agar, 10 wt% pea protein, and 10 wt% malt extract in water. This scaffold was inoculated with *Aspergillus oryzae* by only placing spores on the exterior planes of the scaffold. Inoculation conditions were at 31°C, 80% relative humidity for 45 hours. Through the use of this inoculation technique, the mycelium grew from the surface into the scaffold. Through this guided growth, the mycelium formed an anisotropic mycelium matrix, therefore creating a meat-like texture with this approach. A close-up of the three-dimensional scaffold with mycelium is presented to demonstrate that mycelia growth direction led to an anisotropic structure.

### Example 8

A three-dimensional scaffold was formed by mixing 2wt% agar, 4% Cremodan^{®}, 1% malt sugar, and 3% yeast extract with water and heating to 95°C. After heating, the edible matrix (which may also be referred to as a "food matrix" in the context of the present application) was foamed by using a kitchen aid to reach a high void fraction of 80%. The foamed edible matrix was poured into a mould and slowly cooled down to induce agar gelation. During this cooling step, the fungal mycelia of *Aspergillus oryzae* was stirred into the foamed edible matrix, referred to as inoculation. After cooling, the gel was additionally perforated with sharp needles, rendering a porous network with a large open area, referred to as inoculated three-dimensional scaffold. After this cooling step, the inoculated scaffold was incubated at 31°C and 85% relative humidity. After incubation over 40h, a mycelium network was formed around and in the entire food matrix, rendering a fungus-based food product.

### Example 9

An edible matrix with 2wt% agar, 10% pea protein and 10% malt sugar, was mixed in water and heated to 95°C. After heating, the food matrix was foamed by using a kitchen aid while cooling down to room temperature. This foam was then poured into a mould with spikes and slowly cooled down. Through the spikes in the mould a porous three-dimensional matrix was formed. During this cooling step, the fungal mycelia of *Aspergillus oryzae* was added while cooling down, referred to as inoculation, resulting an inoculated three-dimensional edible scaffold. After this cooling step, the inoculated scaffold was incubated at 31°C and 85% relative humidity. After growth over 40h, a mycelium network was formed around and in the entire food matrix, rendering a meat analogue.

### Example 10

A food matrix with 4% Cremodan^{®}, 1% malt sugar, 3% yeast extract was produced by heating to 95°C and cooling down to 40°C. At 40°C the mycelia spores of *Rhizopus oligosporus* were added into the foam structure. The foam was inoculated at 32°C. The fermented foam formed a closed porous wet foam. The fungi only grew on the surface and did not penetrate into the matrix itself.

### Example 11

Just as in Example 9, a three-dimensional scaffold was formed and inoculated with fungi. However, here the fungi were only inoculated from the top surface, allowing to form directed fungal growth across the voids and thus a directed fungal network, as shown in Figure 8.

### Example 12:

An edible matrix composed of water with 2 wt% agar, 12 wt% pea protein isolate, and 10 wt% sugar, was mixed and heated to boiling point. After heating, the food matrix was cooled to 55°C and spores of *A. oryzae* were added and the edible matrix was foamed by filling 400g of the edible matrix in a 1L pressurized vessel, adding gaseous N₂O or CO₂ to reach a pressure of 6-10 bar, followed by shaking for 10 min. The slurry was released from the vessel leading to an expansion of the gas upon release from the gas pressurized vessel to reach a gas volume fraction of 50-60 vol%. This slurry was transferred into a mould and cooled down to room temperature (20-25°C). After cooling, the matrix formed an inoculated three-dimensional scaffold. Additional voids were provided in the resulting scaffold by perforation with needles with a diameter of around 1 mm. After this perforation step, the inoculated scaffold was incubated at 31°C and 85% relative humidity, either with or without the mould. After growth of 40h, a mycelium network was formed around and in the entire food matrix, rendering a fungus-based food product.

### Example 13:

An edible matrix composed of water with 2 wt% agar, 12 wt% pea protein isolate, and 10 wt% sugar was mixed and heated to boiling point. After heating the food matrix was cooled to 55°C, spores of *A. oryzae* were added and the food matrix was foamed by using a pressurized vessel according to Example 12. This foam was then poured into a mould and slowly cooled down to room temperature. After cooling, the gel formed an inoculated three-dimensional scaffold. Additional voids were provided in the resulting scaffold by perforation with needles with a diameter of around 0.5 mm. After this perforation step, the inoculated scaffold was incubated at 31°C and 85% relative humidity. After growth of 40h, a mycelium network was formed around and in the entire food matrix, rendering a fungus-based food product.

### Example 14.

An edible matrix composed of water with 2 wt% agar, 3 wt% pea protein isolate, 8 wt% pea protein concentrate, and 10 wt% sugar, was mixed and heated to boiling point. The final mixture was lowered to a pH of 5 with a 80% lactic acid solution. After heating, the food matrix was foamed by using a pressurized vessel according to Example 12 while cooling down to room temperature. During this cooling step, the fungal mycelia of *Aspergillus oryzae* was added, referred to as inoculation. This foam was then poured into a mould and slowly cooled down to room temperature. After cooling, the gel formed an inoculated three-dimensional scaffold. Additional voids were provided in the resulting scaffold by perforation with needles with a diameter of around 0.5 mm. After these cooling and perforation steps, the inoculated scaffold was incubated at 31°C and 85% relative humidity. After growth of 40h, a mycelium network was formed around and in the entire food matrix, rendering a fungus-based food product.

### Example 15.

An edible matrix composed of water with 2 wt% agar, 8 wt% pea protein isolate, and 10 wt% sugar, was mixed and heated to 95°C. After heating, the food matrix was foamed by using a rotor-stator system (Polytron, 10'000 rpm, 10 minutes) while cooling down to room temperature to reach a gas volume fraction of 25-35 vol%., After 9 minutes of foaming, the fungal mycelia of *Aspergillus oryzae* was added, referred to as inoculation. This foam was then poured into a mould and slowly continued to cool down to room temperature. After cooling, the gel formed an inoculated three-dimensional scaffold. Additional voids were provided in the resulting scaffold by perforation with needles with a diameter of around 0.1 mm. After this perforation step, the inoculated scaffold was incubated at 27°C and 95% relative humidity. After growth of 27h, a mycelium network was formed around and in the entire food matrix, rendering a fungus-based food product.

### Example 16:

An edible matrix composed of water with 2 wt% agar, 6 wt% pea protein isolate, spices, coloring, and 10 wt% sugar, is mixed and heated to boiling temperature. After heating and initial cooling down to 55°C, spores of *A. oryzae* were added. The inoculated edible matrix is foamed by using a pressurized vessel as described in Example 12 while continuing cooling down to 45°C. This foam is then poured into a mould in the shape of a sausage and slowly cooled down to final temperature of 20°C to solidify the foam. After cooling, additional voids were provided in the resulting scaffold by roughening up the surface. After this roughening step, the inoculated scaffold was incubated at 31°C and 85% relative humidity. After growth of 40h, a mycelium network was formed around and in the entire food matrix, rendering a fungus-based food product resembling a sausage (see Figure 9).

### Example 17:

An edible matrix composed of water with 2 wt% agar, 5 wt% pea protein concentrate, 5 wt% pea protein isolate, 5 wt% rapeseed oil, and 10 wt% malt sugar is mixed and heated to boiling temperature. After heating, the food matrix is cooled down to 55°C, spores of *A. oryzae* were added, and the food matrix was foamed by using a pressurized vessel while cooling down towards room temperature as described in Example 12. This foam is poured into a mould and slowly cooled down to room temperature. After cooling, the resulting scaffold was provided with additional voids by perforation with several needles of different sizes ranging from 0.1 mm to 1 cm in diameter. After this perforation step, the inoculated scaffold was incubated at 31°C and 85% relative humidity. After growth of 40h, a mycelium network was formed around and in the entire food matrix, rendering a fungus-based food product.

### Example 18:

An edible matrix composed of water with 2 wt% agar, 10 wt% pea protein, 10 wt% coconut fat, tomato paste and 10 wt% malt sugar was mixed and heated to boiling temperature. After heating, the food matrix is foamed by using a pressurized vessel (see Example 12) while cooled down to 45°C. This foam is then poured into a mould and slowly cooled down to room temperature. After cooling, the resulting scaffold was provided with additional voids by perforation with a needle of 0.1 mm in diameter. After this perforation step, the food matrix was inoculated with spores of *A. oryzae.* The inoculated scaffold was incubated at 31°C and 85% relative humidity. After growth of 40h, a mycelium network was formed around and in the entire food matrix, rendering a fungus-based food product. The fungus-based food product was pan-fried in oil resulting in a juicy sensation upon consumption, as depicted in Figure 10.

### Example 19:

An edible matrix composed of water with 2 wt% agar, 7 wt% pea protein concentrate, 2,5 wt% pea protein isolate, yeast extract, and 10 wt% malt sugar was mixed and heated to boiling temperature. After heating, the food matrix was foamed by using a pressurized vessel while cooling down to 45°C, according to Example 12. This foam was then poured into a mould and slowly cooled down to room temperature. After cooling, the resulting scaffold was provided with additional voids by perforation with a needle of 0.1 mm in diameter. After this perforation step, the scaffold was inoculated with spores of *A. oryzae.* The inoculated scaffold was incubated at 31°C and 85% relative humidity. After growth of 40h, a mycelium network was formed around and in the entire food matrix, rendering a fungus-based food product.

### Example 20:

An edible matrix composed of water with 2 wt% agar, 7 wt% pea protein concentrate, 2,5 wt% pea protein isolate, 10 wt% texturized protein, and 10 wt% malt sugar was mixed and heated to boiling temperature. After heating and cooling down to 55°C spores of *A. oryzae* were added. The food matrix was foamed by using a pressurized vessel, according to Example 12, while cooling down towards to 45°C. This foam was then poured into a mould and slowly cooled down to room temperature. After cooling, the resulting scaffold was removed from the mould and was provided with additional voids by perforation with a needle of 0.1 mm in diameter. After this perforation step, the inoculated scaffold was incubated at 31°C and 85% relative humidity. After growth of 40h, a mycelium network is formed around and in the entire food matrix, rendering a fungus-based food product.

### Example 21:

An edible matrix composed of water with 2 wt% agar, 7 wt% pea protein concentrate, 2,5 wt% pea protein isolate, and 10 wt% native starch is mixed and heated to boiling temperature. After heating and cooling down spores of *A. oryzae* are added, and the food matrix is foamed by using a pressurized vessel while cooling down towards room temperature. This foam is then poured into a mould and slowly cooled down to room temperature. After cooling, the resulting scaffold is made porous by perforation with a needle. After this perforation step, the inoculated scaffold is incubated at 31°C and 85% relative humidity. After growth of 40h, a mycelium network is formed around and in the entire food matrix, rendering a fungus-based food product.

### Example 22:

An edible matrix composed of water with 2 wt% agar, 7 wt% pea protein concentrate, 2,5 wt% pea isolate, and 10 wt% pre-gelatinized starch was mixed and heated to boiling temperature. After heating and cooling down spores of *A. oryzae* were added and the food matrix was foamed by using a pressurized vessel while cooling down to room temperature. This foam was then poured into a mould and slowly cooled down to room temperature. After cooling, the resulting scaffold was removed from the mould and was provided with additional voids by perforation with a needle of 0.1 mm in diameter. After this perforation step, the inoculated scaffold was incubated at 31°C and 85% relative humidity. After growth of 40h, a mycelium network was formed around and in the entire food matrix, rendering a fungus-based food product.

### Example 23:

An edible matrix composed of water with 2 wt% agar, 7 wt% pea protein concentrate, 2,5 wt% pea protein isolate, and 10 wt% pre-gelatinized starch was mixed and heated to boiling temperature. After heating and cooling down to 55°C spores of *A. oryzae* were added and the food matrix was foamed by using a pressurized vessel, according to Example 12, while cooling down to 45°C. This foam is then poured into a mould and slowly cooled down to room temperature. After cooling, the resulting scaffold was removed from the mould and made porous by perforation with a needle of 0.1 mm in diameter. After these cooling and perforation steps, the inoculated scaffold was incubated at 31°C and 85% relative humidity. After growth of 40h, a mycelium network was formed around and in the entire food matrix, rendering a fungus-based food product.

### Example 24:

An edible matrix composed of water with 2 wt% agar, 5 wt% pea protein concentrate, 5 wt% pea protein isolate, and 10 wt% sugar was mixed and heated to boiling temperature. After heating, the food matrix was foamed by using a rotating membrane device while cooled down to 45°C. Foaming was performed with pressurized air, a membrane with pores of 3 micrometer in diameter to reach a gas volume fraction of 30 vol%. During this process, spores of *A. oryzae* were added into the process. This foam was then poured into a mould and slowly cooled down to room temperature. After cooling, the resulting scaffold was provided with additional poresby needle perforation with needle of 0.1 mm in diameter. After these cooling and perforation steps, the inoculated scaffold was incubated at 27°C and 95% relative humidity. After growth of 30h, a mycelium network was formed around and in the entire food matrix, rendering a fungus-based food product.

### Example 25:

An edible matrix composed of water with 27 wt% pea protein isolate, 0.5 wt% baking powder, and 10 wt% starch was mixed and heated in a microwave. The resulting foam was inoculated with spores of *A. oryzae* and incubated at 27°C and 95% relative humidity. After growth of 30h, a mycelium network was formed around and in the entire food matrix, rendering a fungus-based food product.

### Example 26:

An edible matrix composed of water with 20 wt% pea protein isolate, 10 wt% sugar, *Aspergillus oryzae* spores and transglutaminase was mixed and foamed by using a pressurized vessel, according to Example 12 but at room temperature. The foam was then poured into a mould and cooled down to 5°C for 12h. The resulting porous scaffold was provided with additional voids by perforation with needles with diameter of arround 0.5 mm. The inoculated scaffold is incubated at 31°C and 85% relative humidity for 30h.

### Example 27:

An edible matrix composed of water with 2 wt% agar, 10 wt% pea protein isolate and 10 wt% sugar, was mixed and heated to 95°C. After heating, the food matrix was foamed by using a pressurized vessel according to Example 12 and released into a beaker and cooled down to below 55°C. At below 55°C, the fungal mycelia of *Aspergillus oryzae* was added to the foamed and the beaker and mixed by hand with a spatula, referred to as inoculation. At a temperature of above 45°C, this foam was then poured into a mould and slowly continued to cool down. After cooling, the gel formed an inoculated three-dimensional scaffold. The resulting porous scaffold was removed from the mould, the outer layer (1mm in thickness) was cut off to create a more open surface and the scaffold was provided with additional voids by perforation with needles with a diameter of around 0.3 mm. After this perforation step, the inoculated scaffold was incubated at 31°C and 85% relative humidity for 20h. After growth, the matrix was mixed and blended and re-incubated for another 20h. Through this step, the pieces formed through mixing were re-connected by fungal growth and thus they were able to grow through the entire food matrix, rendering a fungus-based food product.

### Example 28:

In another example the properties of the fungus-based food product of Example 14 were demonstrated. The resulting fungus-based food products have a high water and oil uptake capability. In this example the 3D porous edible scaffold had a water content of 75 wt% before fermentation, 68 wt% after fermentation and is able to exceed the original water content up to 87 wt% after soaking.

### Example 29:

In another example, the properties of the fungus-based food product of Example 14 were demonstrated by its juiciness. The juiciness was defined as the total amount of liquid that was chemically bound by the matrix, as opposed to water that can be expelled from the matrix through mechanical force. The unbound liquid is entrapped in the channels of the porous network of the edible matrix. For this experiment, a disk of 3 cm in height and 8 cm in diameter of the fungus-based food product was soaked in water for 1 hour at room temperature to fully saturate the fungus-based food products with water. The fungus-based food product was able to increase its water content by min. 90 wt% of initial weight. Squeezing of the final product by applying a weight of 3 kg onto the cross-section of the disk after soaking led to a decrease of 13 wt% of uptaken water, so the final product was able to bind 77 wt% additional water. Squeezing led to a decrease of water because unbound water, representing the juiciness, is expelled due to the mechanical force.

### Example 30:

In another example, the properties of the fungus-based food product of Example 14 were demonstrated by its juiciness. For this experiment, a disk of 3 cm in height and 8 cm in diameter of the fungus-based food product was soaked in oil for 1 hour at room temperature to fully saturate the fungus-based food product with oil. The fungus-based food product was able to increase its oil content by 42 wt% of initial weight. Squeezing of the final product by applying a weight of 3 kg onto the cross-section of the disk after soaking led to a decrease of 3 wt% of uptaken oil, so the final product was able to bind 39 wt% additional oil. Squeezing led to a decrease of oil because free oil, representing the unbound, is expelled due to the mechanical force.

### Example 31:

In another example, two different foaming techniques and thus pore sizes were compared. The three-dimensional scaffold made according to Example 18, was either foamed by pressure foaming or by whisking/mechanical foaming and then inoculated with *A. oryzae* and incubated for 40h at 30°C and 85% relative humidity. As shown in Figure 11 a), c1) and d1), the pressure-foamed substrate had larger and more open pores and thus led to a higher stability upon frying and cooking and to an overall higher mechanical stability upon mycelial growth compared to the mechanically-foamed substrate in Figure 11 b), c2) and d2) with smaller and less interconnected or open pores.

### Example 32:

In another example, fungus-based food products with 3 different formulations were compared. As shown in Figure 12, one contained 2 wt% agar and 5 wt% rice protein in water (Figure 12, a), one comprised 2 wt% agar, 5 wt% pea protein concentrate and 5 wt% glucose in water (Figure 12, b), and one comprised 2 wt% agar, 15 wt% glucose and 0.2 wt% yeast extract in water. All edible matrices were pressure-foamed, inoculated with *Aspergillus oryzae* (*A. oryzae*) and incubated for 40 hours at 30°C and 85% relative humidity and moulded in cylindral moulds. The fungal mycelium after incubation was the densest in the product containing both pea protein concentrate and sugar (b), as it provided both a carbon source and a nitrogen source, while instead with little nitrogen (in c), the growth was fast but not dense. As a result, the fungus-based food product in Figure 12 b) showed best shape retention after frying and boiling in water, compared to a) and c), which decreased majorly in volume.

## Claims

1. A method of making a fungus-based food product, said method comprising the steps of:
(a) providing a three-dimensional edible scaffold comprising an edible matrix, the three-dimensional edible scaffold being formed by first providing a continuous edible matrix which is then subsequently converted into the three-dimensional scaffold by introducing voids into the matrix by foaming or puncturing or a combination thereof, wherein the edible matrix comprises micro- and/or macronutrients required for fungal mycelium growth, wherein step (a) does not comprise an extrusion process to create multiple filaments or macrostructures;
(b) inoculating the three-dimensional edible scaffold with at least one fungus to generate an inoculated scaffold; and
(c) incubating the inoculated scaffold at growth conditions that allow for mycelium growth of the at least one fungus so that the at least one fungus grows through the scaffold as a mycelium to form a fungus-based food product.

2. The method of claim 1, wherein the three-dimensional edible scaffold comprises a multitude of said voids which are partly interconnected so as to allow mycelium growth into the scaffold and through it.

3. The method of claim 1 or 2, wherein voids are each bound by at least a concave surface of the scaffold, preferably a concave inner surface of the scaffold.

4. The method of any of the preceding claims,
wherein the fungal mycelium at the end of the incubation step (c) is contained in at least 10% of said voids, more preferably at least 50% and particularly preferred at least 90% of the voids, and/or
said voids being configured to allow to create a global fiber-like texture across the whole food product, and/or
wherein an average size of the voids is between 20 µm to 4 cm, preferably 50 µm to 2 cm, even more preferably 100 µm to 1 cm.

5. The method of any of the preceding claims, wherein the three-dimensional edible scaffold is not formed by assemblying filaments, particularly extruded filaments, preferably the three-dimensional edible scaffold not being formed by printing filaments with voids in between printing lines thereby creating a porous network containing pores and/or channels.

6. The method of any of the preceding claims, wherein the step (a) comprises:
(a1) introducing voids into the edible matrix, preferably by foaming;
(a2) introducing, preferably pouring, the edible matrix into a mould; and
(a3) solidifying the edible matrix in the mould, preferably by gelation,
wherein the edible matrix is introduced into the mould before the edible matrix is formed into a macrostructure,
preferably, wherein distribution of the voids in the scaffold is fixed upon solidification of the edible matrix in the mould.

7. The method of any of the preceding claims, wherein the voids are introduced into the matrix by foaming and the foam is solidified by gelation, preferably the voids are further introduced after gelation by puncturing.

8. The method of any of the preceding claims, wherein in the step (a) the edible matrix is foamed and the foamed edible matrix is poured into a mould and cooled down to induce gelation, preferably agar gelation.

9. The method of any of the preceding claims, wherein the steps (a) and (b) are carried out in one step by inoculating the edible matrix which is used to form the scaffold or by adding the at least one fungus during the formation of the scaffold.

10. The method of any of the preceding claims, wherein the edible scaffold comprises more than 0.1 wt% plant-derived protein and/or saccharide.

11. The method of any of the preceding claims, wherein the edible matrix comprises a gel-forming polysaccharide and/or protein, wherein the gel is formed upon heating, cooling, addition of ions and/or enzymatic cross-linking.

12. The method of any of the preceding claims, wherein the edible scaffold contains at least a certain percentage of components which cannot be consumed by the fungus and which are, therefore, maintained as a scaffold while the fungus is growing into and through the scaffold, preferably the components which cannot be consumed by the fungus comprising agar, carrageenan and/or alginate,
preferably, wherein the three-dimensional edible scaffold comprises fibers, viscosifiers, oils, fats, vitamins, trace elements, enzymes, flavour compounds, colorants, acids, bases and/or salts.

13. The method of any of the preceding claims, wherein said at least one fungus is selected from the group consisting of ascomycetes, basidiomycetes, deuteromycetes, oomycetes, and/or zygomycetes.

14. The method of any of the preceding claims, further comprising the step of:
(d) interrupting the growth of the at least one fungus, preferably by changing the temperature and/or water activity to below or above the temperature and water activity conditions required for growth of the at least one fungus.

15. A fungus-based food product obtainable by the method of any one of claims 1 to 14.

## Patentansprüche

1. Verfahren zur Herstellung eines pilzbasierten Lebensmittelprodukts, wobei das Verfahren die Schritte aufweist:
(a) Bereitstellen eines dreidimensionalen essbaren Gerüsts mit einer essbaren Matrix, wobei das dreidimensionale essbare Gerüst dadurch gebildet wird, dass zunächst eine kontinuierliche essbare Matrix bereitgestellt wird, die dann anschließend in das dreidimensionale Gerüst umgewandelt wird, indem Hohlräume in die Matrix durch Aufschäumen oder Punktieren oder eine Kombination daraus eingebracht werden, wobei die essbare Matrix Mikro- und/oder Makronährstoffe aufweist, die zum Pilzmyzelwachstum erforderlich sind, wobei der Schritt (a) keinen Extrusionsvorgang aufweist, um mehrere Filamente oder Makrostrukturen zu erzeugen;
(b) Inokulieren des dreidimensionalen essbaren Gerüsts mit mindestens einem Pilz, um ein inokuliertes Gerüst zu erzeugen; und
(c) Inkubieren des inokulierten Gerüsts unter Wachstumsbedingungen, die Myzelwachstum des mindestens einen Pilzes ermöglichen, so dass der mindestens eine Pilz durch das Gerüst hindurch als Myzel wächst, um ein pilzbasiertes Lebensmittelprodukt zu bilden.

2. Verfahren nach Anspruch 1, wobei das dreidimensionale essbare Gerüst zahlreiche der Hohlräume aufweist, die teilweise miteinander verbunden sind, um Myzelwachstum in das Gerüst hinein und durch es hindurch zu ermöglichen.

3. Verfahren nach Anspruch 1 oder 2, wobei Hohlräume jeweils durch mindestens eine konkave Oberfläche des Gerüsts begrenzt sind, vorzugsweise eine konkave Innenfläche des Gerüsts.

4. Verfahren nach einem der vorstehenden Ansprüche,
wobei das Pilzmyzel am Ende des Inkubationsschritts (c) in mindestens 10 % der Hohlräume enthalten ist, stärker bevorzugt mindestens 50 % und besonders bevorzugt mindestens 90 % der Hohlräume, und/oder
wobei die Hohlräume so konfiguriert sind, dass sie ermöglichen, eine globale faserartige Textur über das gesamte Lebensmittelprodukt zu erzeugen, und/oder
wobei eine mittlere Größe der Hohlräume zwischen 20 µm und 4 cm, vorzugsweise 50 µm und 2 cm, noch stärker bevorzugt 100 µm und 1 cm liegt.

5. Verfahren nach einem der vorstehenden Ansprüche, wobei das dreidimensionale essbare Gerüst nicht durch Zusammenstellen von Filamenten gebildet wird, insbesondere extrudierten Filamenten, wobei vorzugsweise das dreidimensionale essbare Gerüst nicht durch Drucken von Filamenten mit Hohlräumen zwischen Druckzeilen gebildet wird, wodurch ein poröses Netzwerk erzeugt wird, das Poren und/oder Kanäle enthält.

6. Verfahren nach einem der vorstehenden Ansprüche, wobei der Schritt (a) aufweist:
(a1) Einbringen von Hohlräumen in die essbare Matrix, vorzugsweise durch Aufschäumen;
(a2) Einbringen, vorzugsweise Gießen, der essbaren Matrix in eine Form; und
(a3) Verfestigen der essbaren Matrix in der Form, vorzugsweise durch Gelierung,
wobei die essbare Matrix in die Form eingebracht wird, bevor die essbare Matrix zu einer Makrostruktur ausgebildet wird,
wobei vorzugsweise die Verteilung der Hohlräume im Gerüst bei Verfestigung der essbaren Matrix in der Form fixiert wird.

7. Verfahren nach einem der vorstehenden Ansprüche, wobei die Hohlräume in die Matrix durch Aufschäumen eingebracht werden und der Schaum durch Gelierung verfestigt wird und vorzugsweise die Hohlräume ferner nach Gelierung durch Punktieren eingebracht werden.

8. Verfahren nach einem der vorstehenden Ansprüche, wobei im Schritt (a) die essbare Matrix aufgeschäumt und die aufgeschäumte essbare Matrix in eine Form gegossen und abgekühlt wird, um Gelierung zu induzieren, vorzugsweise Agargelierung.

9. Verfahren nach einem der vorstehenden Ansprüche, wobei die Schritte (a) und (b) in einem Schritt durch Inokulieren der essbaren Matrix, die dazu dient, das Gerüst zu bilden, oder durch Zugeben des mindestens einen Pilzes während der Bildung des Gerüsts durchgeführt werden.

10. Verfahren nach einem der vorstehenden Ansprüche, wobei das essbare Gerüst mehr als 0,1 Gew.-% aus Pflanzen gewonnenes Protein und/oder Saccharid aufweist.

11. Verfahren nach einem der vorstehenden Ansprüche, wobei die essbare Matrix ein Gel-bildendes Polysaccharid und/oder Protein aufweist, wobei das Gel bei Erwärmung, Abkühlung, Zugabe von Ionen und/oder enzymatischer Vernetzung gebildet wird.

12. Verfahren nach einem der vorstehenden Ansprüche, wobei das essbare Gerüst mindestens einen bestimmten Prozentsatz von Komponenten enthält, die nicht vom Pilz verzehrt werden können und die daher als Gerüst beibehalten werden, während der Pilz in das Gerüst hinein und durch es hindurch wächst, wobei vorzugsweise die Komponenten, die nicht vom Pilz verzehrt werden können, Agar, Carrageenan und/oder Alginat aufweisen,
wobei vorzugsweise das dreidimensionale essbare Gerüst Fasern, Eindicker, Öle, Fette, Vitamine, Spurenelemente, Enzyme, Geschmacksverbindungen, Farbstoffe, Säuren, Basen und/oder Salze aufweist.

13. Verfahren nach einem der vorstehenden Ansprüche, wobei der mindestens eine Pilz aus der Gruppe ausgewählt ist, die aus Askomyzeten, Basidiomyzeten, Deuteromyzeten, Oomyzeten und/oder Zygomyzeten besteht.

14. Verfahren nach einem der vorstehenden Ansprüche, ferner mit dem Schritt:
(d) Unterbrechen des Wachstums des mindestens einen Pilzes, vorzugsweise durch Ändern der Temperatur und/oder Wasseraktivität auf unterhalb oder oberhalb der Temperatur- und Wasseraktivitätsbedingungen, die zum Wachstum des mindestens einen Pilzes erforderlich sind.

15. Pilzbasiertes Lebensmittelprodukt, das durch das Verfahren nach einem der Ansprüche 1 bis 14 erreichbar ist.

## Revendications

1. Procédé de production d'un produit alimentaire à base de champignon, ledit procédé comprenant les étapes de :
(a) fourniture d'un échafaudage comestible tridimensionnel comprenant une matrice comestible, l'échafaudage comestible tridimensionnel étant formé tout d'abord par fourniture d'une matrice comestible continue qui est ensuite convertie en l'échafaudage tridimensionnel par introduction de vides dans la matrice par moussage ou perforation ou une combinaison de ceux-ci, dans lequel la matrice comestible comprend des micro- et/ou macro-nutriments requis pour la croissance de mycélium fongique, laquelle étape (a) ne comprend pas de traitement d'extrusion pour créer de multiples filaments ou macrostructures ;
(b) inoculation de l'échafaudage comestible tridimensionnel avec au moins un champignon pour générer un échafaudage inoculé ; et
(c) incubation de l'échafaudage inoculé dans des conditions de croissance qui permettent la croissance de mycélium de l'au moins un champignon de façon que l'au moins un champignon croisse à travers l'échafaudage sous la forme d'un mycélium pour former un produit alimentaire à base de champignon.

2. Procédé selon la revendication 1, dans lequel l'échafaudage comestible tridimensionnel comprend une multitude desdits vides qui sont partiellement interconnectés de façon à permettre la croissance de mycélium dans l'échafaudage et à travers celui-ci.

3. Procédé selon la revendication 1 ou 2, dans lequel des vides sont liés chacun par au moins une surface concave de l'échafaudage, de préférence une surface intérieure concave de l'échafaudage.

4. Procédé selon l'une quelconque des revendications précédentes,
dans lequel le mycélium fongique à la fin de l'étape d'incubation (c) est contenu dans au moins 10 % desdits vides, mieux encore au moins 50 % et tout particulièrement au moins 90 % des vides, et/ou
lesdits vides étant configurés pour permettre la création d'une texture analogue à des fibres globale sur tout le produit alimentaire, et/ou
dans lequel la taille moyenne des vides est comprise entre 20 µm et 4 cm, de préférence entre 50 µm et 2 cm, mieux encore entre 100 µm et 1 cm.

5. Procédé selon l'une quelconque des revendications précédentes, dans lequel l'échafaudage comestible tridimensionnel n'est pas formé par assemblage de filaments, en particulier de filaments extrudés, de préférence l'échafaudage comestible tridimensionnel n'étant pas formé par impression de filaments avec des vides entre lignes d'impression créant ainsi un réseau poreux contenant des pores et/ou canaux.

6. Procédé selon l'une quelconque des revendications précédentes, dans lequel l'étape (a) comprend :
(a1) l'introduction de vides dans la matrice comestible, de préférence par moussage ;
(a2) l'introduction, de préférence le versement, de la matrice comestible dans un moule ; et
(a3) la solidification de la matrice comestible dans le moule, de préférence par gélification,
dans lequel la matrice comestible est introduite dans le moule avant que la matrice comestible ne soit formée en une macrostructure,
de préférence dans lequel la distribution des vides dans l'échafaudage est fixée suite à la solidification de la matrice comestible dans le moule.

7. Procédé selon l'une quelconque des revendications précédentes, dans lequel les vides sont introduits dans la matrice par moussage et la mousse est solidifiée par gélification, de préférence les vides sont en outre introduits après gélification par perforation.

8. Procédé selon l'une quelconque des revendications précédentes, dans lequel, dans l'étape (a), la matrice comestible est expansée et la matrice comestible expansée est versée dans un moule et refroidie pour que soit induite une gélification, de préférence une gélification à la gélose.

9. Procédé selon l'une quelconque des revendications précédentes, dans lequel les étapes (a) et (b) sont mises en œuvre en une seule étape par inoculation de la matrice comestible qui est utilisée pour former l'échafaudage ou par addition de l'au moins un champignon pendant la formation de l'échafaudage.

10. Procédé selon l'une quelconque des revendications précédentes, dans lequel l'échafaudage comestible comprend plus de 0,1 % en poids de saccharide et/ou de protéine d'origine végétale.

11. Procédé selon l'une quelconque des revendications précédentes, dans lequel la matrice comestible comprend une protéine et/ou un polysaccharide filmogène, dans lequel le gel est formé suite à un chauffage, un refroidissement, une addition d'ions et/ou une réticulation enzymatique.

12. Procédé selon l'une quelconque des revendications précédentes, dans lequel l'échafaudage comestible contient au moins un certain pourcentage de composants qui ne peuvent pas être consommés par le champignon et qui sont par conséquent maintenus sous la forme d'un échafaudage alors que le champignon croît dans et à travers l'échafaudage, de préférence les composants qui ne peuvent pas être consommés par le champignon comprenant la gélose, la carraghénane et/ou un alginate,
de préférence dans lequel l'échafaudage comestible tridimensionnel comprend des fibres, des agents conférant une viscosité, des huiles, des graisses, des vitamines, des oligo-éléments, des enzymes, des composés aromatisants, des colorants, des acides, des bases et/ou des sels.

13. Procédé selon l'une quelconque des revendications précédentes, dans lequel ledit au moins un champignon est choisi dans le groupe constitué par les ascomycètes, les basidiomycètes, les deutéromycètes, les oomycètes et/ou les zygomycètes.

14. Procédé selon l'une quelconque des revendications précédentes, comprenant en outre l'étape de :
(d) interruption de la croissance de l'au moins un champignon, de préférence par changement de la température et/ou de l'activité de l'eau à des valeurs inférieures ou supérieures aux conditions de température et d'activité de l'eau requises pour la croissance de l'au moins un champignon.

15. Produit alimentaire à base de champignon pouvant être obtenu par le procédé de l'une quelconque des revendications 1 à 14.
